(19) Europäisches Patentamt
European Patent Office
Office européen des brevets

(11) **EP 2 484 284 A1**

(12) **EUROPEAN PATENT APPLICATION**
published in accordance with Art. 153(4) EPC

(43) Date of publication:
**08.08.2012 Bulletin 2012/32**

(51) Int Cl.:
*A61B 5/1455* (2006.01)     *B60K 28/06* (2006.01)
*G08G 1/14* (2006.01)     *G08G 1/16* (2006.01)

(21) Application number: **09850015.0**

(22) Date of filing: **02.10.2009**

(86) International application number:
**PCT/JP2009/005133**

(87) International publication number:
**WO 2011/039824 (07.04.2011 Gazette 2011/14)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(71) Applicant: **Hochiki Corporation**
**Shinagawa-ku, Tokyo 141-8660 (JP)**

(72) Inventor: **NAGASHIMA, Tetsuya**
**Tokyo 141-8660 (JP)**

(74) Representative: **Tischner, Oliver**
**Lavoix Munich**
**Bayerstrasse 85a**
**80335 Munich (DE)**

(54) **ETHYL ALCOHOL DETECTING DEVICE**

(57) The ethyl alcohol-detecting device of the present invention includes an ethyl alcohol-detecting portion that calculates an ethyl alcohol content corresponding to an ethyl alcohol concentration, based on a first subject image stored in a memory and a reference image stored in a nonvolatile memory; an interfering substance-detecting portion that calculates a degree of interference corresponding to the concentration of an interfering substance, based on a second subject image stored in the memory and the reference image stored in the nonvolatile memory; and a determination portion that determines that ethyl alcohol has been detected when the ethyl alcohol content is equal to or higher than a first predetermined threshold and the degree of interference is less than a second predetermined threshold, and determines that ethyl alcohol has not been detected when the ethyl alcohol content is equal to or higher than the first predetermined threshold and the degree of interference is equal to or higher than the second predetermined threshold.

FIG. 1

EP 2 484 284 A1

**Description**

Technical Field

The present invention relates to an ethyl alcohol-detecting device for determining whether a driver is driving under the influence of alcohol or the like by detecting ethyl alcohol that is dissolved in the blood due to drinking, based on infrared radiated from the human body.

Background Art

Conventionally, as a device for preventing driving under the influence of alcohol, a device is known which detects ethyl alcohol contained in a driver's breath by a gas sensor and prevents the car from being started (for example, see Patent Document 1).
Generally, the concentration of alcohol contained in breath is proportional to blood alcohol concentration. The standard of driving under the influence of alcohol prescribed by law is a breath alcohol concentration of 0.15 mg/L, which corresponds to a blood alcohol concentration of 0.03%. Therefore, by measuring the breath alcohol concentration, it is possible to prevent a driver from driving by detecting the condition of the driver under the influence of alcohol.

Citation List

Patent Literature

[Patent Document 1] Japanese Unexamined Patent Application, First Publication No. H08-150853

Summary of Invention

Technical Problem

However, this type of device for preventing driving under the influence of alcohol in the related art indirectly detects alcohol through a gas sensor, by sucking in the air in the space around the top of the driver's head using a fan. Accordingly, compared to a case where the breath is directly collected to measure the alcohol concentration, the measured value becomes lower than an actual breath alcohol concentration, which leads to a concern that driving under the influence of alcohol cannot be reliably prevented.
Moreover, since the sensitivity of the gas sensor for detecting alcohol is changed due to foreign substances being attached, oxidation, or the like, the sensor needs a calibration process for automatically adjusting sensitivity. In addition, there is a problem in that the sensor cannot maintain detection accuracy unless maintenance such as regular cleaning inspection is provided.
The present invention has been made in consideration of the above circumstances, and an object thereof is to provide a maintenance-free ethyl alcohol-detecting device that is usable for preventing driving under the influence of alcohol or the like by detecting ethyl alcohol in the blood from infrared radiated from a driver.

Solution to Problem

The present invention employs the following configurations to solve the above problems and accomplish the above object.

(Device Using Infrared Imaging Element)

(1) The ethyl alcohol-detecting device of the present invention includes an imaging element that has sensitivity to an infrared wavelength band; an optical system that forms an image of a subject on the imaging element; a memory and a nonvolatile memory that store the image captured by the imaging element; a first filter that selectively transmits infrared light of a first wavelength band including an absorption wavelength of ethyl alcohol released from the subject; a second filter that selectively transmits infrared light of a second wavelength band including a wavelength which is another absorption wavelength of an interfering substance having an absorption wavelength as same as that of the ethyl alcohol released from the subject in the first wavelength band and is a wavelength other than the absorption wavelength of the ethyl alcohol; an imaging control unit that controls the imaging element to capture a first subject image formed by passing through the first filter and a second subject image formed by passing through the second filter and stores the images in the memory; a reference image-registering unit that stores the first subject image in the nonvolatile memory as a reference image, at the time of setting a registration mode; an ethyl alcohol-detecting portion that calculates an ethyl alcohol content corresponding to the ethyl alcohol concentration, based on the first subject image stored in the memory and the reference image stored in the nonvolatile memory; an interfering substance-detecting portion that calculates a degree of interference corresponding to the concentration of the interfering substance, based on the second subject image stored in the memory and the reference image stored in the nonvolatile memory; and a determination portion that determines that ethyl alcohol has been detected when the ethyl alcohol content is equal to or higher than a first predetermined threshold and the degree of interference is less than a second predetermined threshold, and determines that ethyl alcohol has not been detected when the ethyl alcohol content is equal to or higher than the first predetermined threshold and the degree of interference is equal to or higher than the second predetermined threshold.

(2) The ethyl alcohol-detecting device according to section (1) may employ a configuration wherein the first filter selectively transmits infrared light in a wavelength band including 2.77 $\mu$m or 3.37 $\mu$m as the first wavelength band; and the second filter selectively transmits infrared light in a wavelength band including 3.28 $\mu$m as the second wavelength band when the interfering substance is menthol, and selectively transmits infrared light in a wavelength band including 5.88 $\mu$m as the second wavelength band when the interfering substance is stearic acid.

(3) The ethyl alcohol-detecting device according to section (1) may employ a configuration wherein the reference image-registering unit calculates a reference amount of light received as the sum or average of the amounts of light received by respective pixels constituting the reference image and stores the reference amount of light received in the nonvolatile memory; the ethyl alcohol-detecting portion calculates the ethyl alcohol content based on an amount of light received by the first subject that is calculated as the sum or average of the amounts of light received by respective pixels of the first subject image and the reference amount of light received which is stored in the nonvolatile memory; and the interfering substance-detecting portion calculates the degree of interference based on an amount of light received by the second subject that is calculated as the sum or average of the amounts of light received by respective pixels of the second subject image and the reference amount of light received which is stored in the nonvolatile memory.

(4) The ethyl alcohol-detecting device according to section (3) may employ a configuration wherein the ethyl alcohol-detecting portion calculates the ethyl alcohol content as (i) a subtracted value that is obtained by subtracting the amount of light received by the first subject from the reference amount of light received, or a divided value that is obtained by dividing the reference amount of light received by the amount of light received by the first subject, (ii) a value obtained by multiplying the subtracted value or the divided value by a first predetermined coefficient, or (iii) the sum or average of squared error values of the amount of light received by the first subject and the reference amount of light received; and the interfering substance-detecting portion calculates the degree of interference as (iv) a subtracted value obtained by subtracting the amount of light received by the second subject from the reference amount of light received or a divided value obtained by dividing the reference amount of light received by the amount of light received by the second subject, (v) a value obtained by multiplying the subtracted value or the divided value by a second predetermined coefficient, or (vi) the sum or average of squared error values of the amount of light received by the second subject and the reference amount of light received.

(Device Using Infrared-Detecting Element)

(5) Another ethyl alcohol-detecting device of the present invention includes an infrared sensor that has at least one infrared light-receiving element; an optical system that forms an image of a subject on the infrared light-receiving sensor; a memory and a nonvolatile memory that store a light-reception signal received by the infrared sensor; a first filter that selectively transmits infrared light of a first wavelength band including an absorption wavelength of ethyl alcohol released from the subject; a second filter that selectively transmits infrared light of a second wavelength band including a wavelength which is another absorption wavelength of an interfering substance having an absorption wavelength as same as that of the ethyl alcohol released from the subject in the first wavelength band and is a wavelength other than the absorption wavelength of the ethyl alcohol; a light-reception control portion that detects a first light-reception signal of the subject passing through the first filter and received by the infrared sensor and a second light-reception signal of the subject passing through the second filter and received by the infrared sensor, and stores the signals in the memory; a reference light-reception signal registering portion that stores the first light-reception signal in the nonvolatile memory as a reference light-reception signal, at the time of setting a registration mode; an ethyl alcohol-detecting portion that calculates an ethyl alcohol content corresponding to the ethyl alcohol concentration, based on the first light-reception signal stored in the memory and the reference light-reception signal stored in the nonvolatile memory; an interfering substance-detecting portion that calculates a degree of interference corresponding to the concentration of the interfering substance, based on the second light-reception signal stored in the memory and the reference light-reception signal stored in the nonvolatile memory; and a determination portion that determines that ethyl alcohol has been detected when the ethyl alcohol content is equal to or higher than a first predetermined threshold and the degree of interference is less than a second predetermined threshold, and determines that ethyl alcohol has not been detected when the ethyl alcohol content is equal to or higher than the first predetermined threshold and the degree of interference is equal to or higher than the second predetermined threshold.

(6) The ethyl alcohol-detecting device according to section (5) may employ a configuration wherein the first filter selectively transmits infrared light in a wavelength band including 2.77 μm or 3.37 μm as the first wavelength band; and the second filter selectively transmits infrared light in a wavelength band including 3.28 μm as the second wavelength band when the interfering substance is menthol, and selectively transmits infrared light in a wavelength band including 5.88 μm as the second wavelength band when the interfering substance is stearic acid.

(7) The ethyl alcohol-detecting device according to section (5) may employ a configuration wherein the ethyl alcohol-detecting portion calculates the ethyl alcohol content based on the first light-reception signal stored in the memory and the reference light-reception signal stored in the nonvolatile memory; and the interfering substance-detecting portion calculates the degree of interference based on the second light-reception signal stored in the memory and the reference light-reception signal stored in the nonvolatile memory.

(8) The ethyl alcohol-detecting device according to section (7) may employ a configuration wherein the ethyl alcohol-detecting portion calculates the ethyl alcohol content as (i) a subtracted value that is obtained by subtracting the first light-reception signal from the reference light-reception signal, or a divided value that is obtained by dividing the reference light-reception signal by the first light-reception signal, (ii) a value obtained by multiplying the subtracted value or the divided value by a first predetermined coefficient, or (iii) the sum or average of squared error values of the first light-reception signal and the reference light-reception signal; and the interfering substance-detecting portion calculates the degree of interference as (iv) a subtracted value obtained by subtracting the second light-reception signal from the reference light-reception signal or a divided value obtained by dividing the reference light-reception signal by the second light-reception signal, (v) a value obtained by multiplying the subtracted value or the divided value by a second predetermined coefficient, or (vi) the sum or average of squared error values of the second light-reception signal and the reference light-reception signal.

(9) In the ethyl alcohol-detecting device according to section (5), the infrared sensor may further include a sensor case that accommodates a combination of the first filter, at least a sheet of the second filter, and the infrared light-receiving element.

Advantageous Effects of Invention

According to the ethyl alcohol-detecting device of the present invention, infrared light radiated from the human body (particularly, infrared light radiated from the face where the skin is exposed) due to the body temperature of a subject (driver) is imaged, and based on this imaged infrared light radiated, a characteristic absorption spectrum created by ethyl alcohol in the subject's capillaries is detected. Consequently, it is possible to detect an ethyl alcohol content corresponding to the blood alcohol concentration. Therefore, whether the subject (driver) is driving under the influence of alcohol or the like is accurately determined based on the ethyl alcohol content, whereby it is possible to take appropriate actions such as calling attention, preventing driving, and the like for the subject.

Menthol contained in cosmetics is an interference factor since this substance has an absorption spectrum as the same wavelength as that of ethyl alcohol. However, menthol also has an absorption spectrum at a wavelength that is intrinsic to menthol and not found in ethyl alcohol. Accordingly, by detecting the absorption spectrum at a wavelength intrinsic to menthol as a degree of interference, it is possible to reliably prevent false detection of ethyl alcohol resulting from cosmetics and the like.

In the present invention, the ethyl alcohol content is detected as a ratio or a difference between the amount of light received in the absorption spectrum at a wavelength intrinsic to ethyl alcohol and the amount of light received at another wavelength not having the same absorption spectrum. Accordingly, it is possible to accurately detect ethyl alcohol in the subject's (driver's) capillaries with a high S/N ratio, without being influenced by the magnitude of the amount of infrared radiation.

Moreover, in the present invention, the ethyl alcohol content is detected from the absorption spectrum of ethyl alcohol in the infrared light radiated from the subject. Accordingly, there is no necessity to provide maintenance such as sensitivity adjustment or cleaning inspection as in a case of alcohol detection performed by a gas sensor used in the related art. Therefore, it is possible to detect the ethyl alcohol content in the human body, which is a criterion for determining the drive under the influence of alcohol, with a stable, high accuracy for a long time.

Furthermore, in the present invention, as a reference subject image that is used when measuring the ethyl alcohol content or the degree of interference, a subject image of an absorption wavelength of ethyl alcohol that is obtained by imaging the subject at the time when ethyl alcohol is not contained in the subject's blood is used. This subject image is obtained in a registration mode at the beginning of use of the device, and registered in advance in a nonvolatile memory. Consequently, there is no necessity to continually capture subject images of different wavelength bands where the absorption spectrum of ethyl alcohol does not exist, as reference images. Therefore, a subject image may be obtained regarding two wavelength bands including the absorption wavelength of ethyl alcohol and the absorption wavelength intrinsic to menthol or the like which becomes an interfering factor. As a result, it is possible to simplify an optical system that images an infrared image of a subject and the switching control thereof, and to reduce costs.

Brief Description of Drawings

FIG. 1 is a block diagram showing an ethyl alcohol-detecting device according to a first embodiment of the present invention.
FIG. 2 is a transparent perspective view illustrating an infrared camera used in the embodiment.
FIG. 3 is a graph illustrating a wavelength spectrum distribution of ethyl alcohol, wherein the horizontal axis indicates the wavelength, and the vertical axis indicates the amount of light received.
FIG. 4 is a view illustrating the extraction of a characteristic area in the embodiment.
FIG. 5 is a flowchart showing an ethyl alcohol detection process in the embodiment.
FIG. 6 is a flowchart showing the detail of a registration process of the amount of light received by reference image in step S 1 of FIG. 5.
FIG. 7 is a perspective view showing the infrared camera that uses a filter-switching unit used in the embodiment.
FIG. 8 is a block diagram showing an ethyl alcohol-detecting device using an infrared sensor according to a second embodiment of the present invention.
FIG. 9 is a perspective view showing the infrared sensor of FIG. 8.
FIG. 10 is a flowchart showing an ethyl alcohol detection process in the embodiment.
FIG. 11 is a flowchart showing the detail of a registration process of a reference light-reception signal in step S33 of FIG. 10.
FIG. 12 is a block diagram showing an ethyl alcohol-detecting device using a multi-infrared sensor according to a third embodiment of the present invention.
FIG. 13 is a perspective view showing the infrared sensor of FIG. 12.
FIG. 14 is a flowchart showing an ethyl alcohol detection process in the embodiment.
FIG. 15 is a flowchart showing the detail of a registration process of a reference light-reception signal in step S54 of FIG. 14.
FIG. 16 is a block diagram showing an ethyl alcohol-detecting device using a multi-infrared sensor including a built-in filter according to a fourth embodiment of the present invention.
FIG. 17 is a perspective view showing the infrared sensor of FIG. 16.
FIG. 18 is a flowchart showing an ethyl alcohol detection process in the embodiment.
FIG. 19 is a flowchart showing the detail of a registration process of a reference light-reception signal in step S81 of FIG. 18.

Description of Embodiments

The respective embodiments of the ethyl alcohol-detecting device of the present invention will be described below. In the following description, the same constitutional elements are marked with the same reference signs to omit repetitive description.

[First Embodiment]

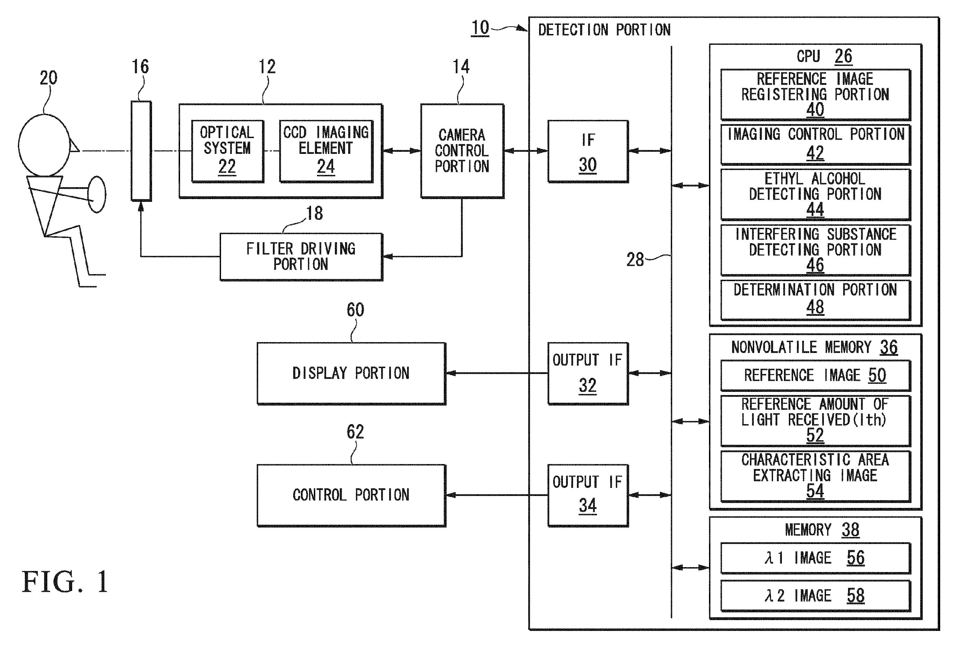

FIG. 1 is a block diagram showing the first embodiment of the ethyl alcohol-detecting device of the present invention, which is mounted in a vehicle.
As shown in FIG. 1, the ethyl alcohol-detecting device of the present embodiment is provided with a detection portion 10 that performs an ethyl alcohol detection process, an infrared camera 12 that includes an optical system 22 and a CCD imaging element 24, a camera control portion 14 that controls the infrared camera 12, a wavelength-variable filter 16 that is disposed between a driver (subject) 20 and the infrared camera 12, and a filter-driving portion 18 that drives the wavelength-variable filter 16. In addition, the detection portion 10 is provided with a CPU 26 that representatively indicates the hardware environment of a computer.

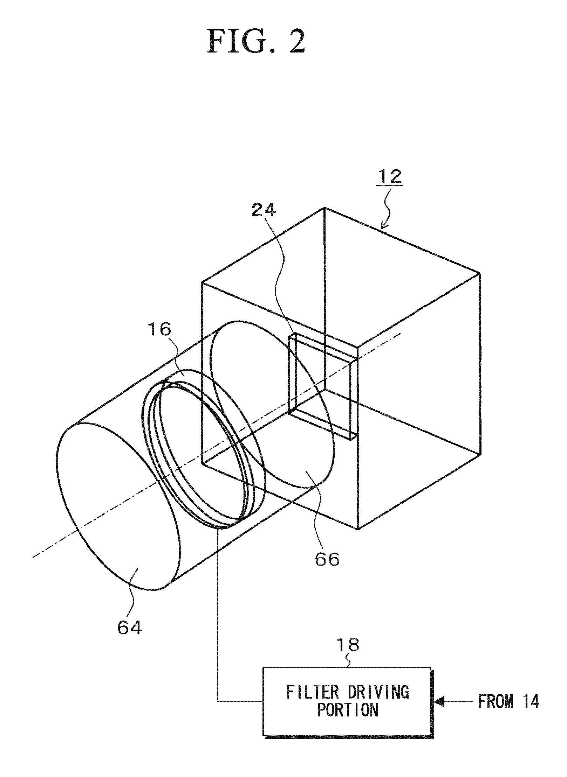

FIG. 2 is a transparent perspective view of the infrared camera 12 that uses the wavelength-variable filter 16 used in the present embodiment.

As shown in FIG. 2, the optical system 22 of the infrared camera 12 is provided with an object lens 64, an imaging lens 66, and the wavelength-variable filter 16 that is disposed between the object lens 64 and the imaging lens 66. A transmission wavelength of the wavelength-variable filter 16 is controlled by the filter-driving portion 18.

In the infrared camera 12, the CCD imaging element 24 having sensitivity to an infrared wavelength band is disposed. An infrared image of a wavelength band passing through the wavelength-variable filter 16 is formed on the CCD imaging element 24 by the imaging lens 66, and the CCD imaging element 24 captures this image.

The wavelength-variable filter 16 is a Fabry-Perot interference filter, and has, for example, a pair of glass substrates which has a thickness of about 200 angstroms to 300 angstroms and is deposited on a surface facing a metallic permeable film that serves as a reflective film of Au or the like. The pair of glass substrates is arranged such that they face each other while interposing a piezoelectric element therebetween, and there is a minute gap between the substrates. The piezoelectric element disposed between the pair of glass substrates receives a DC voltage that is applied from the filter-driving portion 18, thereby varying the gap between the pair of glass substrates. When light is incident from one of the pair of glass substrates, the wavelength-variable filter 16 transmits the light covering a plurality of transmission spectra, due to an interference action caused by multiple reflection occurring between the permeable metallic films. As this type of wavelength-variable filter 24, for example, a filter disclosed in Japanese Unexamined Patent Application, First Publication No. H08-285688 can be used.

As shown in FIG. 1, the CPU 26 provided in the detection portion 10 is connected to an interface 30 (hereinafter, referred to as an IF 30), output interfaces 32 and 34 (hereinafter, referred to as output IFs 32 and 34), a nonvolatile memory 36 using a flash memory or EEPROM, and a memory 38 using RAM, via a bus 28.

The IF 30 is connected to the camera control portion 14. When there is a request for ethyl alcohol detection, for example, when the engine of a vehicle is started, the camera control portion 14 converts an image of the driver 20, which is captured by operating the wavelength-variable filter 16 and the infrared camera 12, into digital image data, and then stores the image as a $\lambda1$ image 56 and a $\lambda2$ image 58 in the memory 38. The operation for controlling image capturing using the infrared camera 12 is performed by an imaging control unit 42 provided in the CPU 26.

The output IF 32 is connected to a display portion 60, and the output IF 34 is connected to a control portion 62. In the display portion 60, as a detection result processed in the detection portion 10, for example, whether the driver 20 is under the influence of alcohol is displayed. The display portion 60 also has an audio output function. When the detection portion 10 determines that the driver 20 is driving the car under the influence of alcohol, the control portion 62 performs control for preventing driving under the influence of alcohol by, for example, preventing the engine from being started.

The CPU 26 includes, as functions realized by the execution of a program, a reference image-registering portion 40, the imaging control unit 42, an ethyl alcohol-detecting portion 44, an interfering substance-detecting portion 46, and a determination portion 48.

The detection portion 10 of the present embodiment detects a state where the driver 20 is under the influence of alcohol, based on ethyl alcohol contained and shown in capillaries just beneath the facial skin or the like. The ethyl alcohol contained in the capillaries is detected based on the detection of attenuation of an absorption spectrum at a specific wavelength caused by ethyl alcohol in infrared light that is radiated or reflected from the human body due to the body temperature of the driver 20.

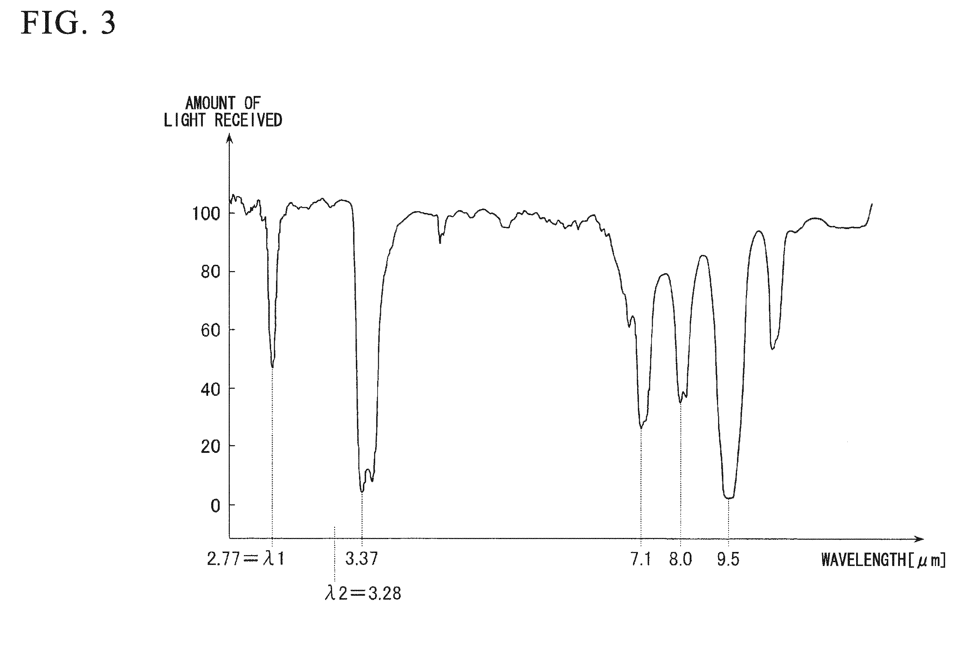

FIG. 3 is a graph showing a wavelength spectrum distribution of ethyl alcohol detected in the present embodiment. The ethyl alcohol $C_2H_5OH$ has an absorption spectrum originating from the molecular structure thereof at a specific wavelength such as 2.77 $\mu$m, 3.37 $\mu$m, or 9.5 $\mu$m. For example, an absorption spectrum at a wavelength of 2.77 $\mu$m is a wavelength band where an ethyl alcohol molecule absorbs light along with the bond stretching of -$CH_3$ in the ethyl alcohol.

Therefore, the infrared light is monitored by controlling the wavelength-variable filter 16 to function as a first filter ($\lambda1$ filter) that selectively transmits a first wavelength band including an absorption wavelength $\lambda1=2.77$ $\mu$m of ethyl alcohol, from infrared light radiated from the driver 20. Then in a state where ethyl alcohol is contained in the capillaries (that is, in a state under the influence of alcohol), the infrared light in the first wave length band including $\lambda1=2.77$ $\mu$m indicates an absorbance according to the ethyl alcohol concentration in the capillaries. At this time, an amount of light received I1 obtained from the respective pixels in the image ($\lambda1$ image) captured by the CCD imaging element 24 is reduced.

In the present embodiment, in a registration mode at the beginning of the use of the ethyl alcohol-detecting device, the $\lambda1$ image that is captured by controlling the wavelength-variable filter 16 to function as the $\lambda1$ filter which includes a wavelength $\lambda1=2.77$ $\mu$m providing an absorption spectrum of ethyl alcohol of a normal time when ethyl alcohol is not contained in the capillaries is stored in the nonvolatile memory 36 as a reference image 50. Subsequently, an amount of light received Ith obtained from the reference image 50 is stored in the nonvolatile memory 36 as a reference amount of light received 52.

Herein, in a state where the ethyl alcohol-detecting device has not yet been used, a reference amount of light received that was determined as a designed value in a manufacturing stage of the ethyl alcohol-detecting device is stored in the nonvolatile memory 36 as a default value $(Ith)_0$. When the registration mode is executed, the default value $(Ith)_0$ is updated to a value Ith intrinsic to a user.

The reference amount of light received Ith obtained from the reference image in the registration mode is an amount of light received corresponding to the amount of infrared radiated (or reflected) from the human body at a normal time when ethyl alcohol is not contained in the body.

Provided that a $\lambda 1$ amount of light received at the time when the driver is drunk and the absorption spectrum created by ethyl alcohol is detected is I1, an ethyl alcohol content A is obtained by the following Formula (1) as a ratio between I1 and the reference amount of light received Ith at a normal time.

$$A=Ith/I1 \qquad (1)$$

The $\lambda 1$ amount of light received I1 of the Formula (1) decreases as the ethyl alcohol concentration increases. Accordingly, the ethyl alcohol content A increases with the ethyl alcohol concentration. In addition, at a normal time, the $\lambda 1$ amount of light received I1 is almost the same as the amount of light received Ith, so the ethyl alcohol content A becomes a value close to A=1.

If the ethyl alcohol content A obtained from the Formula (1) is equal to or higher than a predetermined threshold Ath, it may be possible to determine whether ethyl alcohol has been detected. If the ethyl alcohol content A is less than the threshold, it may be possible to determine whether ethyl alcohol has not been detected. As the threshold Ath, provided that an alcohol content at a normal time is $A_0$, the threshold may be set to $Ath=\alpha A_0$, by multiplying $A_0$ by a predetermined coefficient $\alpha$ that is equal to or greater than 1 so as to absorb an error. For example, provided that $\alpha=1.2$, $Ath=1.2A_0$. A calculation process of the ethyl alcohol content A in the present embodiment is performed by the reference image-registering unit 40 provided in the CPU 26 shown in FIG. 1, the imaging control unit 42, and the ethyl alcohol-detecting portion 44.

When the registration mode in which the ethyl alcohol-detecting device mounted on a vehicle starts to be used is set, the reference image-registering unit 40 controls the camera control portion 14 by the imaging control unit 42 and drives the filter-driving portion 18, thereby switching the wavelength-variable filter 16 to the $\lambda 1$ filter that selectively transmits the first wavelength band including the absorption wavelength $\lambda 1=2.77$ $\mu m$ of ethyl alcohol. Subsequently, the reference image-registering unit 40 captures a subject image that passes through the wavelength-variable filter 16 and is formed on the CCD imaging element 24 as the reference image 50, and stores the image in the nonvolatile memory 36.

The reference image-registering unit 40 calculates the sum or average of the light amounts of light received by the respective pixels constituting the reference image 50 as Ith, and stores the value Ith in the nonvolatile memory 36 as the amount of light received 52.

In a stage where the registration is performed but the device has not yet been used, a reference amount of light received $(Ith)_0$ as a default value determined as a designed value has already been registered in the nonvolatile memory 30. Accordingly, when the registration process executed by the setting of the registration mode is not performed, the reference amount of light received $(Ith)_0$ as the default value is used for the ethyl alcohol detection process.

In addition, the reference image-registering unit 40 creates a characteristic area extracted image 54 for extracting characteristic areas of the driver 20 used for the ethyl alcohol detection, from the reference image 50, and stores this image as a template. The characteristic area extracted image 54 is also used for the extraction of characteristic areas that is performed when a reference amount of light received is obtained from the reference image 50.

The imaging control unit 42 starts to operate when there is a request for the ethyl alcohol detection along with the start of the engine of a vehicle. In addition, while setting the $\lambda 1$ filter by controlling the filter-driving portion 18 to switch the wavelength-variable filter 16 and to switch the wavelength-variable filter 16 to the wavelength band of the $\lambda 2$ filter that will be described later, the imaging control unit 42 controls the infrared camera 12 to capture a facial image of the driver 20. Thereafter, the imaging control unit 42 stores the $\lambda 1$ image 56 as the first subject image formed by passing through the $\lambda 1$ filter and the $\lambda 2$ image 58 as the second subject image formed by passing through the $\lambda 2$ filter in the memory 38.

In the present embodiment, by detecting the characteristics of the absorption spectrum created by ethyl alcohol in the blood, which are included in the infrared radiated from the human body due to the body temperature, the ethyl alcohol content corresponding to the blood alcohol concentration is obtained. However, when the amount of signals of the CCD imaging element 24 is insufficient, it is possible to use an infrared light source as an auxiliary light source.

Reasons for an insufficient amount of signals of the CCD imaging element 24 include conditions limiting camera disposition, for example, a long distance between the infrared camera 12 and the human body, the lens diameter of the infrared camera 12 that cannot be sufficiently increased, and the like. When the infrared light source is used, by detecting the characteristics of a reflection spectrum of the infrared reflected from the human body, which are exhibited due to the ethyl alcohol in the blood, it is possible to obtain the ethyl alcohol content A corresponding to the blood alcohol concentration.

Based on the reference amount of light received 52 (=Ith) obtained from the $\lambda 1$ image 56 stored in the memory 38 and the reference image 50 stored in the nonvolatile memory 36, the ethyl alcohol-detecting portion 44 calculates the ethyl alcohol content A corresponding to the ethyl alcohol concentration from the Formula (1).

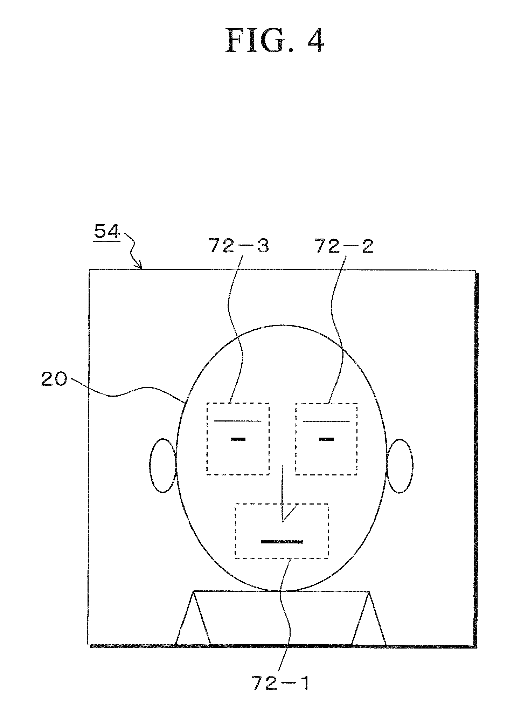

FIG. 4 shows the characteristic area extracted image 54 of the driver that is created by the reference image-registering unit 40 of FIG. 1 and used when the ethyl alcohol content A is calculated in the ethyl alcohol-detecting portion 44. In order to detect the ethyl alcohol concentration in the capillaries of the driver 20 from the amount of infrared light received, it is necessary to calculate the ethyl alcohol content A by specifying a site where the facial capillaries of the driver 20 appear on the skin surface, and the attenuation of an absorption spectrum caused by ethyl alcohol is sufficiently exhibited.

In a case of FIG. 4, during a reference image registration process that is performed in a normal state where ethyl alcohol is not contained in the blood, the characteristic area extracted image 54, where sites have been set from which infrared is easily radiated from the capillaries due to the body temperature, for example, a characteristic-extracting area 72-1 where the skin is thin, such as lips, or characteristic-extracting areas 72-2 and 72-3 around both eyes, is created with respect to the reference image 50 that is obtained by passing through the $\lambda 1$ filter. This characteristic area extracted image 54 is then stored in the nonvolatile memory 36.

If the characteristic area extracted image 54 is successfully created with respect to the driver 20, the reference image-registering unit 40 calculates the reference amount of light received 52, based on the sum or average of the amounts of light received by the respective pixels of the characteristic-extracting areas 72-1 to 72-3 set in the characteristic area-extracted image 54.

In order to calculate the amount of light received I1 of the $\lambda 1$ image 56 for calculating the ethyl alcohol content A in the Formula (1), the ethyl alcohol-detecting portion 44 calculates the sum or average of the amounts of light received by the respective pixels included in the characteristic-extracting areas 72-1 to 72-3 of the characteristic area-extracted image 54, and takes the calculated value as I1. Thereafter, the ethyl alcohol-detecting portion 44 calculates the ethyl alcohol content A from the Formula (1) using Ith which is the value of the reference amount of light received 52 that has already been stored in the nonvolatile memory 36.

In the Formula (1), the value $(I_{th}/I1)$ obtained by dividing the reference amount of light received Ith of the reference image 50 by the amount of light received I1 of the $\lambda 1$ image 56 is used. However, instead of this, a value $(I_{th}-I_1)$ obtained by subtracting the amount of light received I1 of the $\lambda 1$ image 56 from the reference amount of light received Ith of the reference image 50 may be employed, and in addition, a squared value of the divided value $(I_{th}/I_1)^2$ or a square error $(I_{th}-I_1)^2$ may also be employed.

In the present embodiment, based on the $\lambda 2$ image 58 captured by switching the wavelength-variable filter 16 to the wavelength band of the $\lambda 2$ filter, a degree of interference B is calculated by the interfering substance-detecting portion 46, and the propriety of the ethyl alcohol content A calculated by the ethyl alcohol-detecting portion 44 is determined by the determination portion 48.

When the driver 20 as a subject is, for example, a woman, her face to be captured may be wearing makeup. If substances contained in the cosmetics include, for example, menthol $C_{10}H_{20}O$, there is a concern that there will be an absorption spectrum at $\lambda 1=2.77$ $\mu$m in the $\lambda 1$ image 56 with respect to menthol, so the ethyl alcohol content A will be detected falsely, even if there is no ethyl alcohol.

That is, menthol $C_{10}H_{20}O$ contained in cosmetics or the like has an absorption spectrum at 2.77 $\mu$m and 3.37 $\mu$m, similarly to ethyl alcohol. Moreover, menthol $C_{10}H_{20}O$ also has an intrinsic absorption spectrum at a wavelength $\lambda 2=3.28$ $\mu$m originating from the benzene-type structure thereof.

Therefore, in the present embodiment, menthol contained in cosmetics or the like is regarded as an interfering substance in the ethyl alcohol detection, and the wavelength-variable filter 16 is switched to the wavelength band of the $\lambda 2$ filter as the second filter which selectively transmits an intrinsic absorption wavelength $\lambda 2=3.28$ $\mu$m other than the same absorption wavelengths of $\lambda 1=2.77$ $\mu$m and 3.37 $\mu$m as those of ethyl alcohol. Thereafter, an amount of light received I2 is calculated from the captured image of the radiated infrared light passing through the $\lambda 2$ filter, that is, from the $\lambda 2$ image 58. Subsequently, using the reference amount of light received Ith obtained from the reference image 50 from which an amount of light received corresponding to the amount of infrared radiated from the human body in a normal state where ethyl alcohol is not contained in the blood is obtained, the degree of interference B for determining menthol is calculated by the following Formula (2).

$$B=Ith/I2\cdots(2)$$

If the degree of interference B obtained from the Formula (2) is equal to or higher than a predetermined threshold Bth, it is possible to determine that the detected substance is not ethyl alcohol but menthol. The method of determining the threshold Bth is the same as that in the case of the ethyl alcohol content A.

That is, the interfering substance-detecting portion 46 provided in the CPU 26 of FIG. 1 calculates the degree of interference B according to the Formula (2), based on the amount of light received Ith of the reference image 50 obtained by passing through the λ1 filter at a normal time when ethyl alcohol is not contained in the blood and the amount of light received I2 obtained from the λ2 image 58 obtained by passing through the λ2 filter, which have been stored in the nonvolatile memory 36.

Based on the ethyl alcohol content A detected by the ethyl alcohol-detecting portion 44 and the degree of interference B detected by the interfering substance-detecting portion 48, the determination portion 48 determines that the driver is in a state under the influence of alcohol if ethyl alcohol is detected, or determines that the driver is in a normal state if ethyl alcohol is not detected.

Specifically, when the ethyl alcohol content A is equal to or higher than a predetermined threshold Ath, and the degree of interference B is less than a predetermined threshold Bth, the determination portion 46 determines that ethyl alcohol has been detected and that the driver is in a state under the influence of alcohol. On the other hand, when the ethyl alcohol content A is equal to or higher than a predetermined threshold Ath, and the degree of interference B is also equal to or higher than a predetermined threshold Bth, the determination portion 48 determines that ethyl alcohol has not been detected since the above results are yielded not from ethyl alcohol but from an interfering substance (for example, menthol contained in cosmetics or the like), and determines that the driver is in a normal state.

In addition to menthol, the interfering substance in the present embodiment also includes stearic acid $C_{17}H_{35}COOH$ which is widely used for cosmetics or the like just as menthol. The stearic acid has an absorption spectrum at wavelengths of 2.77 μm and 3.37 μm just as ethyl alcohol, but the absorption spectrum of stearic acid also appears at a wavelength of 5.88 μm which results from -COOH that ethyl alcohol does not have.

Accordingly, for the stearic acid, a λ3 filter is prepared as a third filter by switching the wavelength-variable filter 16 such that the wavelength-variable filter 16 selectively transmits a wavelength λ3=5.88 μm, and in this state, a λ3 image captured by the infrared camera 12 is stored in the memory 38 (the λ3 image is not shown in FIG. 1).

In the process performed on the λ3 image corresponding to the absorption spectrum of stearic acid by the interfering substance-detecting portion 46, an amount of light received I3 is calculated from the λ3 image, and in the same manner as in the case of the λ2 image, the degree of interference B is calculated from the Formula (2) with respect to the stearic acid. If the degree of interference B is equal to or higher than the predetermined threshold Bth, the determination portion 48 determines that the substance is not ethyl alcohol but stearic acid.

When the interfering substance with respect to ethyl alcohol is glycerin $C_3H_5(OH)_3$, a wavelength of 2.77 μm of the absorption spectrum originating from -CH3 does not appear. Accordingly, regarding the glycerin, the attenuation of the amount of light received I1 does not occur in the λ1 image 56 of the λ1 filter, and the ethyl alcohol content A calculated from the Formula (1) is equal to or higher than the predetermined threshold Ath. Consequently, it is possible to confirm that the substance is not ethyl alcohol. Therefore, there is no necessity to consider glycerin as an interfering substance.

In the present embodiment, menthol and stearic acid contained in cosmetics or the like in many cases are exemplified as interfering substances. However, when there are substances that exhibit spectral absorption at the same wavelength as that of ethyl alcohol in addition to menthol and stearic acid, regarding wavelengths intrinsic to the respective substances excluding the absorption wavelength of ethyl alcohol, images created by filters intrinsic to the wavelengths are obtained so as to calculate the degree of interference, whereby it is possible to reliably avoid false detection of ethyl alcohol resulting from the interfering substance.

There are countless substances that contain -CH3 and -OH. Among these, menthol and stearic acid contained in cosmetics were exemplified, as substances that are likely to be positively applied to the face. In addition to these substances, acetoacetate generated in the body of a diabetic patient also contains -CH3 and -OH and has the same spectral absorption band as that of ethanol. However, by measuring in advance an absorption band at 5.83 μm that ethanol does not have as the third wavelength band, it is possible to perceive the acetoacetate as an interfering substance.

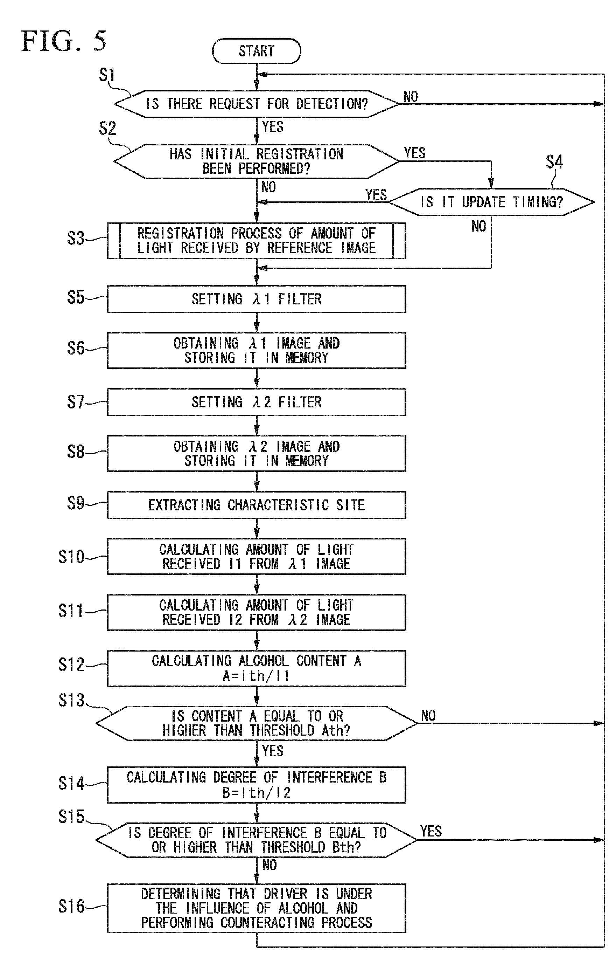

FIG. 5 is a flowchart showing an ethyl alcohol detection process in the present embodiment. As shown in FIG. 5, first, in step S1, whether there is a request for ethyl alcohol detection is checked. Regarding this detection request, for example, when the driver 20 takes a seat and turns an ignition key to the "ON" position to start the engine, a detection request signal is output to the detection portion 10, whereby the detection process is started.

When it is determined that there is a detection request in step S1, the process proceeds to step S2, and whether or not the initial registration of reference image 50, the reference amount of light received 52, and the characteristic area extracted image 54 has been performed in the nonvolatile memory 36 is checked.

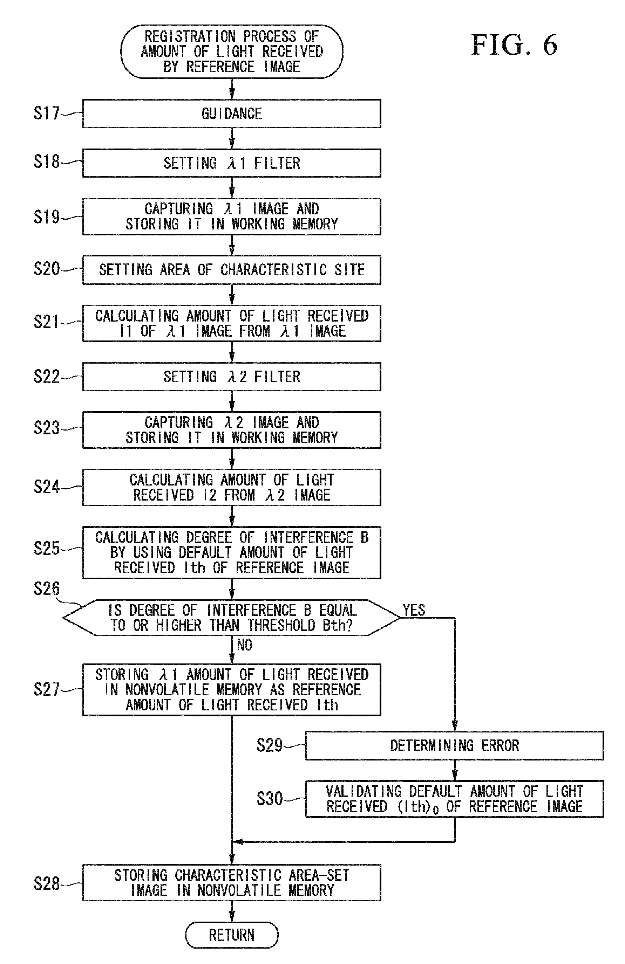

When the user uses the delivered car for the first time, the initial registration has not yet been performed. In this case, the process proceeds to step S3. In step S3, a registration process of the amount of light received by a reference image is executed by the reference image-registering unit 40. The detail of the registration process of the amount of light received by a reference image of step S3 is illustrated in the flowchart of FIG. 6.

If it is determined that the initial registration has been performed in step S2, the process proceeds to step S4. In step S4, whether or not the current point of time is an update timing is checked. As the update timing, for example, one month is set as a cycle. Whenever one month elapses, it is determined that it is update timing in step S4, so the process proceeds to step S3 such that the registration process of the amount of light received by the reference image is executed again and the content of the pervious registration is updated.

Thereafter, in step S5, the wavelength-variable filter 16 is set to the wavelength band of the $\lambda1$ filter. Subsequently, in step S6, the infrared camera 12 is operated to capture an image, and the $\lambda1$ image 56 is obtained and stored in the memory 38. At this time, when an infrared light source is provided in the infrared camera 12, the infrared light source is turned on so as to subsidiarily emit the infrared light to the driver 20, and the $\lambda1$ image 56 is obtained by the reflected light and stored.

Subsequently, in step S7, the wavelength-variable filter 16 is set to the band of the $\lambda2$ filter, and in step S8, the $\lambda2$ image 58 is stored in the memory 38 by the imaging operation performed.

The storage of the $\lambda1$ image 56 and the $\lambda2$ image 58 in the memory in steps S5 to S8 may be performed once. Alternatively, if necessary, these images may be continuously captured and stored a plurality of times.

Thereafter, in step S9, the characteristic area extracted image 54 that has been stored in the nonvolatile memory 36 and includes the characteristic-extracting areas 72-1 to 72-3 in which sites where heat from the capillaries is easily indicated, such as lips and eyes of the driver 20, have been set as shown in FIG. 4 is used so as to be set in the $\lambda1$ image 56 and the $\lambda2$ image 58 stored in the memory 38, whereby the amounts of light received by the pixels included in the characteristic-extracting areas 72-1 to 72-3 are calculated.

First, in step S 10, the amount of light received I1 is calculated from the $\lambda1$ image 56, and then in step S11, the light-reception I2 is calculated from the $\lambda2$ image.

Thereafter, in step S 12, the ethyl alcohol content A is calculated from the Formula (1). Then in step S 13, whether or not the calculated ethyl alcohol content A is equal to or higher than the threshold Ath is checked. If the ethyl alcohol content A is equal to or higher than the threshold Ath, it is determined that ethyl alcohol is contained in the capillaries, and the process proceeds to step S 14. On the other hand, if the ethyl alcohol content A is less than the threshold Ath, since there is no spectral absorption caused by alcohol, it is determined that ethyl alcohol has not been detected, and the process returns to step S1.

In step S 14 to which the process proceeds when the ethyl alcohol content A is equal to or higher than the threshold Ath, the degree of interference B is calculated from the Formula (2). In the subsequent step S 15, it is determined whether or not the calculated degree of interference B is equal to or higher than the threshold Bth. When it is determined that the degree of interference B is equal to or higher than the threshold Bth, this is determined as a false detection of the ethyl alcohol content A caused by an interfering substance other than ethyl alcohol, and the driver is regarded to be in a normal state where ethyl alcohol is not detected, so the process returns to step S1.

On the other hand, when the degree of interference B is less than the threshold Bth in step S 15, it is determined that this is not a false detection of ethyl alcohol caused by an interfering substance, and the process proceeds to step S16. In step S16, it is determined that the driver is in a state under the influence of alcohol since ethyl alcohol has been detected, and a process for counteracting this state is executed.

As the counteracting process executed when the driver is determined to be in a state under the influence of alcohol, a sign of caution for preventing the driver from driving under the influence of alcohol is output and displayed on the display portion 60 shown in FIG. 1, or an operation for prohibiting driving is performed by the control portion 62 such that the engine is not started even if the ignition key is turned to the start position, thereby preventing the driver from driving under the influence of alcohol.

FIG. 6 is a flowchart showing the detail of a registration process of the amount of light received by the reference image in step S3 of FIG. 5.

For the registration process of the amount of light received by the reference image in FIG. 6, first, in step S 17, guidance for performing the registration process is output using the display portion 60 or an audio output function. As this guidance, for example, a message of "a driver's reference image will be registered, please put your hands on the handle without applying cosmetics and face forward, an image will be captured." is output.

Subsequently, in step S 18, the wavelength-variable filter 16 is set to the band of the $\lambda1$ filter. Then the infrared camera 12 performs the imaging operation in step S19, whereby the $\lambda1$ image is stored in the memory 38 used as a working memory.

Next, in step S20, the characteristic area extracted image 54 in which the characteristic-extracting areas 72-1 to 72-3 including the lips or eyes of the driver 20 have been set as shown in FIG. 4 is created with respect to the $\lambda1$ image stored in the memory 38, and the created image is stored in the memory 38.

Subsequently, In step S21, the characteristic area extracted image 54 created in step S20 is set with respect to the $\lambda1$ image in the memory 38, and the amount of light received I1 of the $\lambda1$ image is calculated as the sum or average of the amounts of light received by the respective pixels included in the characteristic-extracting areas 72-1 to 72-3.

Thereafter, a determination process is performed for determining whether or not an interfering substance such as menthol exists on the face of the driver 20 to be registered due to cosmetics. This process is performed by setting the wavelength-variable filter 16 to the band of the $\lambda 2$ filter in step S22, and storing the $\lambda 2$ image in the memory 38 used as a working memory by means of the imaging operation of the infrared camera 12 in step S23.

Next, in step S24, using the characteristic area extracted image 54 created with respect to the $\lambda 2$ image in step S20, the amount of light received I2 of the $\lambda 2$ image is calculated as the sum or average of the amounts of light received by the respective pixels included in the characteristic-extracting areas 72-1 to 72-3.

Subsequently, in step S25, the degree of interference B is calculated using the Formula (2), from the default reference amount of light received $(Ith)_0$ stored in advance in the nonvolatile memory 32 and the $\lambda 2$ amount of light received I2 calculated in step S24.

Thereafter, in step S26, if the calculated degree of interference B is less than the threshold Bth, it is determined that the face of the driver 20 as a target of initial registration is not wearing makeup, and that the facial image is appropriate for the initial registration, and the process proceeds to step S27. In the subsequent step S27, the amount of light received I1 of the $\lambda 1$ image stored in the memory 38 is stored in the nonvolatile memory 36 as the reference amount of light received Ith.

In step S28, the $\lambda 1$ image stored in step S19 and the characteristic area-extracted image obtained in step S20 are stored as the reference image 50 and the characteristic area extracted image 54 respectively in the nonvolatile memory 36.

On the other hand, if the degree of interference B is equal to or higher than the threshold Bth in step S26, the face of the driver 20 as a target of the initial registration is wearing makeup, and the amount of light received I1 of the $\lambda 1$ image to be a reference amount of light received has been calculated with respect to the image having an absorption spectrum in the $\lambda 1$ image showing the absorption wavelength of ethyl alcohol. Consequently, in this case, in step S29, it is determined that there is an error. Thereafter, in step S30, the default reference amount of light received $(Ith)_0$ stored in advance in the nonvolatile memory 36 is validated, and the reference amount of light received Ith depending on the driver 20 is not updated by the registration process. Even when it is determined that there is an error, the characteristic area extracted image 54 is stored in the nonvolatile memory 36 in step S28.

By the registration process of an amount of light received by the reference image as shown in FIG. 6, it is possible to correctly obtain the reference amount of light received Ith intrinsic to the driver 20, which is obtained from the $\lambda 1$ image in a normal state where ethyl alcohol is not contained blood and used as a reference. In addition, in the subsequent use of the device, the correct reference amount of light received Ith in accordance with the change such as the physical condition of the driver is obtained by an update process of the registration of the amount of light received by the reference image, whereby it is possible to guarantee the reliability of the ethyl alcohol-detecting device.

In addition, even when the registration process of the amount of light received by the reference image fails, it is possible to use a default reference amount of light received prepared as a value set in the manufacturing stage. Accordingly, although the accuracy in detecting ethyl alcohol is slightly inferior compared to the reference amount of light received that depends on the driver 20, even if the registration process of the amount of light received by the reference image fails, it is basically possible to effectively perform the ethyl alcohol detection process using the default reference amount of light received $(Ith)_0$.

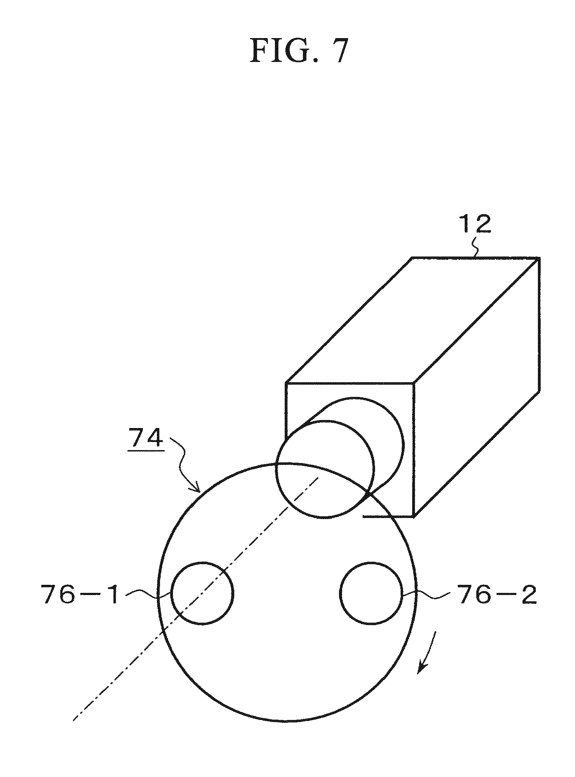

FIG. 7 is a perspective view showing the filter-switching type infrared camera 12 using a rotating disk that is used instead of the wavelength-variable filter 16 shown in FIG. 1.

The infrared camera 12 is provided with the optical system 22 including the object lens 64 and the imaging lens 66, and the CCD imaging element 24, as shown in FIG. 2. Between the driver 20 and the infrared camera 12, a filter-switching unit 74 as a wavelength-variable filter is disposed.

That is, in front of the infrared camera 12, the filter-switching unit 74 is disposed instead of the wavelength-variable filter 16 shown in FIG. 1. A $\lambda 1$ filter 76-1 and a $\lambda 2$ filter 76-2 differing in a band are mounted on, for example, two sites in the rotating disk of the filter-switching unit 74. While these $\lambda 1$ filter 76-1 and $\lambda 2$ filter 76-2 are switched in order by rotating the rotating disk by means of a filter-driving portion (not shown) including a motor, an image created by infrared light radiated or reflected from the face of the driver 20 face is captured.

[Second Embodiment]

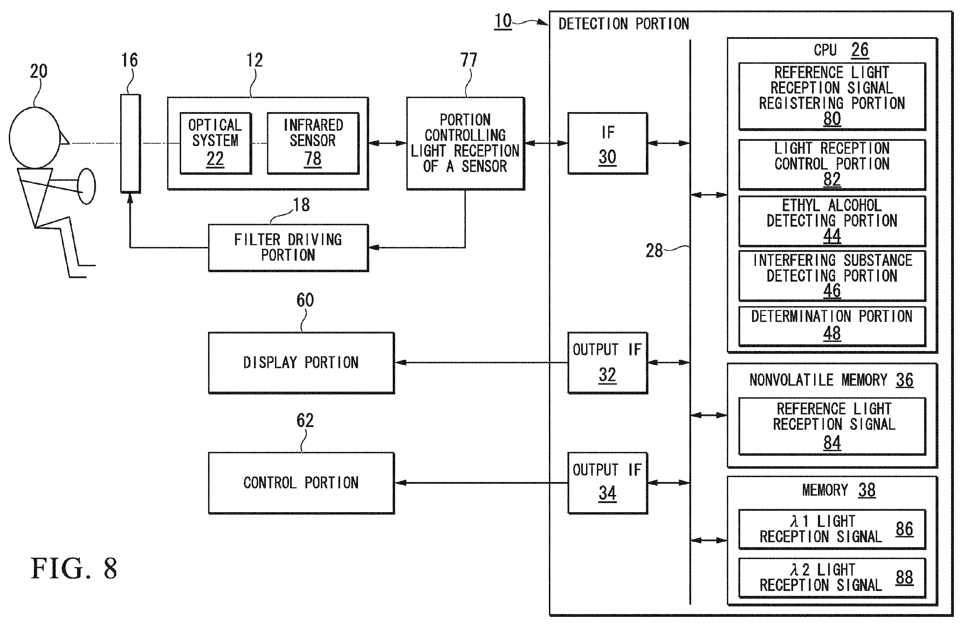

FIG. 8 is a block diagram showing the second embodiment of the ethyl alcohol-detecting device of the present invention, which is mounted on a vehicle.

In FIG. 8, the infrared camera 12 provided in association with the detection portion 10 includes an infrared sensor 78 instead of the CCD imaging element 24 in the first embodiment.

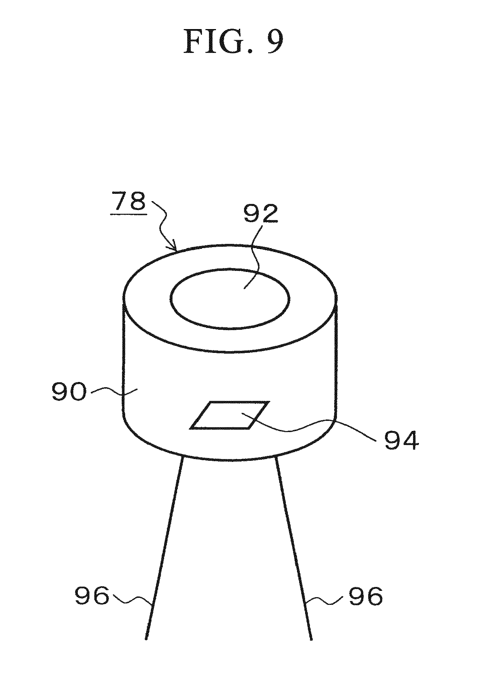

FIG. 9 is a perspective view of the infrared sensor 78. This infrared sensor 78 includes a window 92 of glass or the like in the opening portion of a case 90, and inside the window 92, an infrared-detecting element 94 is provided so as to be shown in a transparent state. The infrared-detecting element 94 is connected to external leads 96.

As the infrared-detecting element 94, non-cooling type elements such as a pyroelectric element, a thermopile, a thermistor, and a bolometer are used. In addition, cooling type elements such as MCT and Insb may also be used.

As shown in FIG. 8, the infrared sensor 78 is provided in the infrared camera 12, and a portion 77 controlling light-reception of a sensor is provided instead of the camera control portion 14 in the first embodiment. This portion 77 controlling light-reception of a sensor drives the filter-driving portion 18, thereby setting the band of the wavelength-variable filter 16.

In addition, in the CPU 26 of the detection portion 10, a reference light-reception signal registering portion 80 and a light-reception control portion 82 are provided in association with the infrared sensor 78. The ethyl alcohol-detecting portion 44, the interfering substance-detecting portion 46, and the determination portion 48 provided in the CPU 26 are the same as those in the first embodiment.

The reference light-reception signal registering portion 80 operates at the beginning of the initial use of the vehicle. Moreover, the reference light-reception signal registering portion 80 stores a light-reception signal detected by the infrared sensor 78 of the infrared camera 12 in the nonvolatile memory 36 as a reference light-reception signal 84 (of which the value is Ith), in a state where the wavelength-variable filter 16 is set to the $\lambda1$ filter including $\lambda1=2.77$ $\mu$m which is the absorption wavelength of ethyl alcohol due to the operation of the filter-driving portion 18 caused by the light-reception control portion 82.

In a state where the wavelength-variable filter 16 has been set to the $\lambda1$ filter, the light-reception control portion 82 stores a $\lambda1$ light-reception signal 86 detected by the infrared sensor 78 of the infrared camera 12 in the memory 38. In addition, in a state where the wavelength-variable filter 16 has been set to the $\lambda2$ filter, the light-reception control portion 82 stores a $\lambda2$ light-reception signal 88 obtained by the infrared sensor 78 in the memory 38.

That is, when the infrared sensor 78 is employed, by the light-reception control performed along with the switching of the filter band caused by the wavelength-variable filter 16, the memory 38 can directly obtain I1 as a $\lambda1$ amount of light received 86 and I2 as a $\lambda2$ amount of light received 88 respectively.

Consequently, in the ethyl alcohol-detecting portion 44, the ethyl alcohol content A is calculated by the Formula (1), from a reference light-reception signal Ith stored in advance in the nonvolatile memory 36 and the $\lambda1$ amount of light received I1 stored in the memory 38.

In addition, the interfering substance-detecting portion 46 calculates the degree of interference B by the Formula (2), from the reference light-reception signal Ith stored in the nonvolatile memory 36 and the $\lambda2$ light-reception signal I2 stored in the memory 38. Moreover, the determination portion 48 can finally determine whether or not ethyl alcohol has been detected, from the ethyl alcohol content A and the degree of interference B.

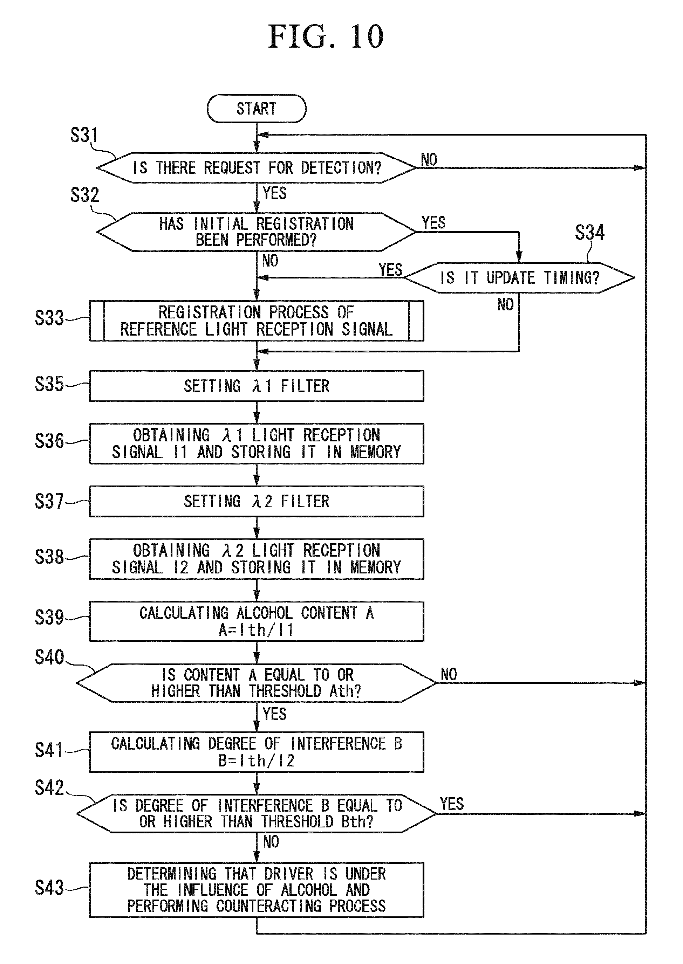

FIG. 10 is a flowchart showing the ethyl alcohol detection process in the present embodiment.

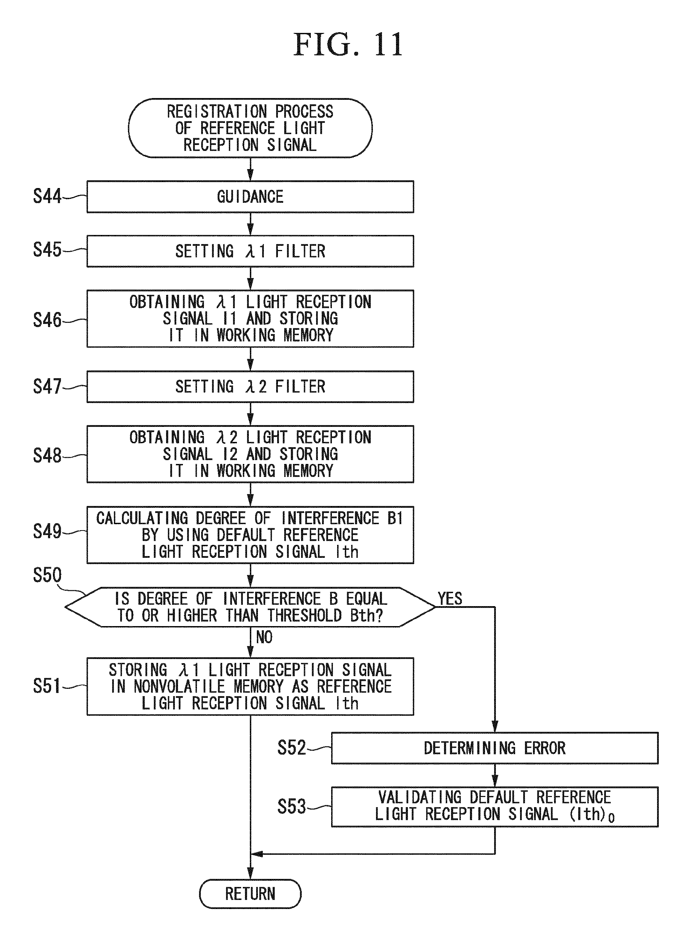

As shown in FIG. 10, after whether there is a request for detection is determined in step S31, the process proceeds to step S32 so as to check whether or not the initial registration has been performed. When the initial registration has not yet been performed, the process proceeds to step S33 to execute the registration process of the reference light-reception signal. The detail of the registration process of the reference light-reception signal will be shown in the flowchart of FIG. 11.

When it is determined that the initial registration has been performed in step S32, whether or not the current point of time is an update timing is checked in step S34. For example, if the current point of time is an update timing of a monthly cycle, the registration process of the reference light-reception signal is executed again in step S33, thereby updating the reference light-reception signal. If the current point of time is not an update timing, the process of step S33 is skipped, and the process proceeds to step S35.

In the subsequent step S35, the wavelength-variable filter 16 is set to the $\lambda1$ filter. Then in step S36, the $\lambda1$ light-reception signal I1 is obtained from the infrared sensor 78 of the infrared camera 12 and stored in the memory 38. In the subsequent step S37, the wavelength-variable filter 16 is set to the $\lambda2$ filter. Then in step S38, the $\lambda2$ light-reception signal I2 is obtained from the infrared sensor 78 and stored in the memory 38.

In the subsequent step S39, the ethyl alcohol content A is calculated by the Formula (1). Next, in step S40, if the content A is less than the threshold Ath, it is determined that ethyl alcohol has not been detected, and the process returns to step S31. On the other hand, if the content A is equal to or higher than the threshold Ath, the process proceeds to step S41, and a degree of interference B1 is calculated by the Formula (2).

In the subsequent step S42, it is determined whether or not the degree of interference B is equal to or higher than the threshold Bth. If the degree of interference B is equal to or higher than the threshold Bth, the content A is not the alcohol content A obtained by ethyl alcohol but the alcohol content A obtained by an interfering substance such as menthol. Accordingly, it is determined that ethyl alcohol has not been detected, and the process proceeds to step S31. On the other hand, if the degree of interference is determined to be less than the threshold Bth in step S42, it is determined that ethyl alcohol has been detected. Accordingly, it is determined that the driver is in a state under the influence of alcohol in step S43, and a process for counteracting this state is performed.

FIG. 11 is a flowchart showing the detail of the registration process of the reference light-reception signal of step S33 in FIG. 10.

As shown in FIG. 11, in the registration process of the reference light-reception signal, guidance is provided to the driver as display or audio in step S44 so as to perform the registration process of the reference light-reception signal. Thereafter, the wavelength-variable filter 16 is set to the $\lambda1$ filter in step S45, and the $\lambda1$ light-reception signal I1 is obtained by the infrared sensor 78 in step S46 and stored in the memory 38.

In the subsequent step S47, the wavelength-variable filter 16 is set to the $\lambda 2$ filter, and in step S48, the $\lambda 2$ light-reception signal I2 is obtained from the infrared sensor 78 and stored in the memory 38 as a working memory. Next, in step S49, the degree of interference B is calculated by the Formula (2), using the default reference light-reception signal Ith stored in advance in the nonvolatile memory 36.

In the subsequent step S50, when the degree of interference B is less than the threshold Bth, the driver 20 who is not wearing makeup is in a state appropriate for the registration process of the reference light-reception signal. Therefore, in step S51, the $\lambda 1$ light-reception signal I1 in step S46 stored in the memory 38 is stored in the nonvolatile memory 36 as the reference light-reception signal Ith.

On the other hand, when the degree of interference B is equal to or higher than the threshold Bth in step S50, the face of the driver 20 to be registered is wearing makeup, and the ethyl alcohol absorption spectrum is created by an interfering substance. Consequently, in step S52, it is determined that there is an error. Thereafter, in step S53, the default reference light-reception signal $(Ith)_0$ stored in advance in the nonvolatile memory 36 is validated so as to make it possible to calculate the ethyl alcohol content A or the degree of interference B using the default reference light-reception signal $(Ith)_0$ when the process returns to the main routine of FIG. 10.

As described above, even when the infrared sensor 78 is employed in the infrared camera 12, the reference light-reception signal Ith used for calculating the ethyl alcohol content A and the degree of interference B can be set based on an actual light-reception signal of the infrared radiated from the face of the driver 20 or the infrared reflected when an infrared light source is used. Therefore, by using the reference light-reception signal depending on the driver 20, it is possible to further heighten the accuracy of the ethyl alcohol detection process.

[Third Embodiment]

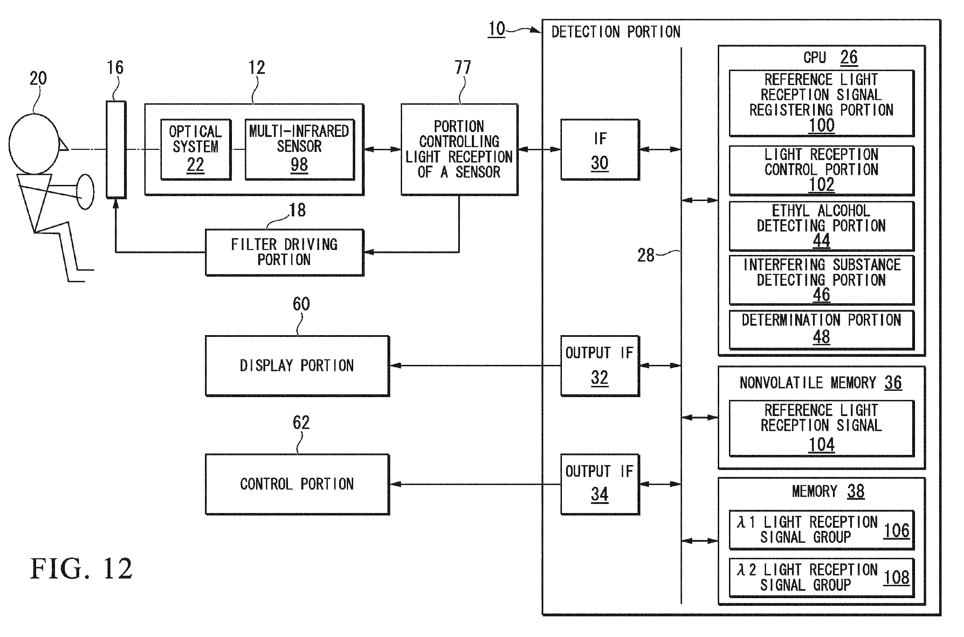

FIG. 12 is a block diagram showing the third embodiment of the ethyl alcohol-detecting device of the present invention, which is mounted on a vehicle.
As shown in the FIG. 12, in the infrared camera 12 of the present embodiment, a multi-infrared sensor 98 is provided as well as the optical system 22.

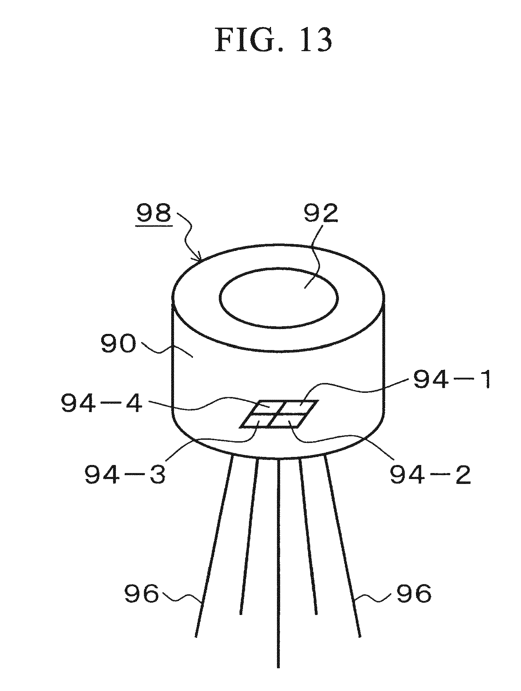

FIG. 13 is a perspective view of the multi-infrared sensor 98 shown in FIG. 12. In the multi-infrared sensor 98, for example, four infrared-detecting elements 94-1 to 94-4 are arranged such that the multi-infrared sensor 98 is shown transparently inside a case 90 where the window 92 is provided on a light-reception side.

Consequently, when the image of a subject which is the driver 20 is formed on the multi-infrared sensor 98, the light-reception signal of the subject image (that is, the facial image of the driver 20) is obtained by each of four divided areas (each area corresponding to each of the infrared-detecting elements 94-1 to 94-4). Accordingly, by calculating the sum or average of the light-reception signals received by each of the quartered infrared-detecting elements 94-1 to 94-4, it is possible to obtain the $\lambda 1$ amount of light received I1 and the $\lambda 2$ amount of light received I2.

Moreover, during the registration process performed by a reference light-reception signal registering portion 100, the sum or average of the four light-reception signals obtained from the quartered infrared-detecting elements 94-1 to 94-4, and the thus obtained $\lambda 1$ light-reception signal I1 is taken as the reference amount of light received Ith. This process is performed by a reference light-reception signal registering portion 100 and a light-reception control portion 102 that are provided in the CPU 26 of FIG. 12 in association with the multi-infrared sensor 98. The configuration other than these is the same as that of the second embodiment which uses a single infrared-detecting element shown in FIG. 8.

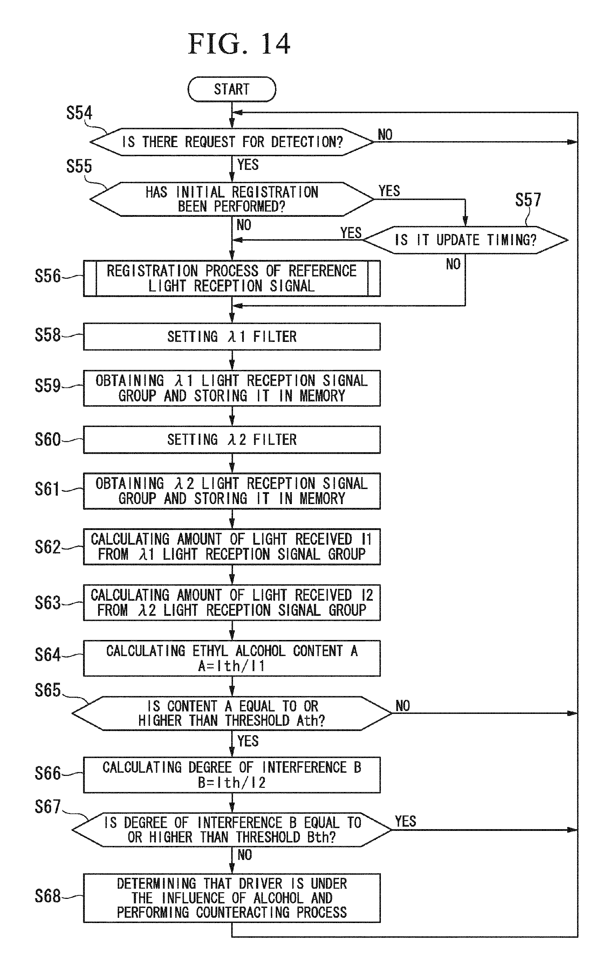

FIG. 14 is a flowchart showing the ethyl alcohol detection process in the present embodiment.
As shown in FIG. 14, steps S54 to S57 are the same as steps S31 to S34 shown in FIG. 10. In addition, steps S64 to S68 of FIG. 14 are the same as steps S39 to S43 shown in FIG. 10.

The present embodiment is different from the second embodiment in that the calculation of the amount of light received in steps S58 to S63 of FIG. 14 is based on the light-reception signals of the four infrared-detecting elements 94-1 to 94-4 shown in FIG. 13. In other words, the wavelength-variable filter 16 is set to the $\lambda 1$ filter in step S58, and a $\lambda 1$ light-reception signal group 106 consisting of four light-reception signals from the infrared-detecting elements 94-1 to 94-4 of the multi-infrared sensor 98 is obtained and stored in the memory 38 in step S59.

In the subsequent step S60, the wavelength-variable filter 16 is set to the $\lambda 2$ filter. Next, in step S61, a $\lambda 2$ light-reception signal group 108 consisting of four light-reception signals from the infrared-detecting elements 94-1 to 94-4 of the multi-infrared sensor 98 is obtained and stored in the memory 38.

Thereafter, in step S62, the amount of light received I1 is calculated as the sum or average of the four light-reception signals from the $\lambda 1$ light-reception signal group 106. Likewise, in step S63, the amount of light received I2 is calculated as the sum or average of the four signals of the $\lambda 2$ light-reception signal group 108.

As described above, by obtaining the $\lambda 1$ light-reception signal group 106 and the $\lambda 2$ light-reception signal group 108 from the sum or average of a plurality of light-reception signals using, for example, the multi-infrared sensor 98 which includes four infrared-detecting elements 94-1 to 94-4, it is possible to further heighten the detection sensitivity and the resolution of infrared, compared to a case where the single infrared-detecting element 94 shown in FIG. 9 is used. In addition, the number of the infrared-detecting elements may be increased as necessary, such as 8 elements, 16 elements, or 32 elements, in addition to 4 elements.

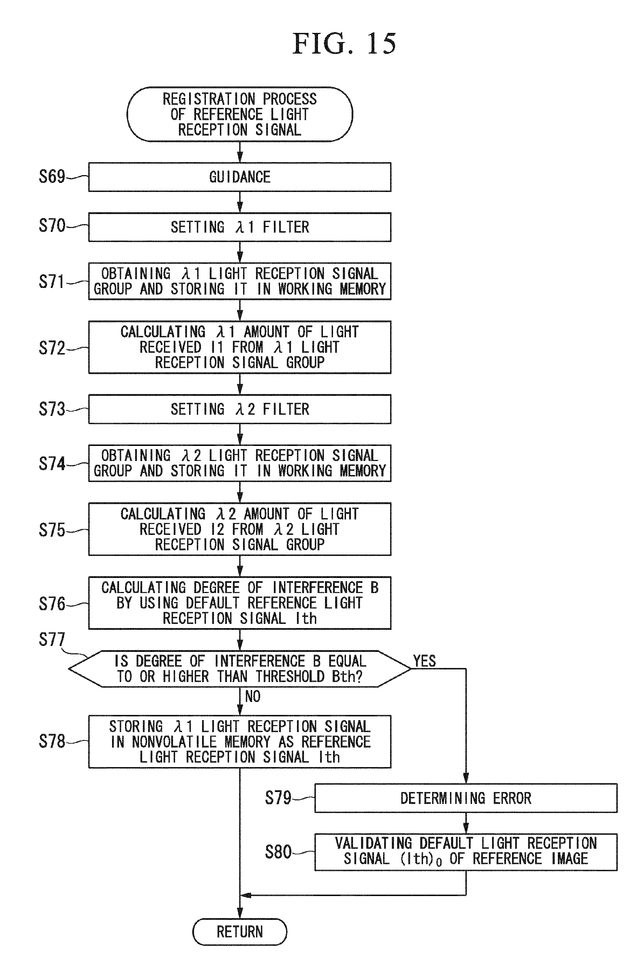

FIG. 15 is a flowchart showing the detail of the registration process of the reference light-reception signal in step S56 of FIG. 14.

This registration process of the reference light-reception signal includes steps S69 to S80, and is basically the same as the process in the case where the single infrared-detecting element 94 shown in FIG. 11 is used. However, the registration process of the reference light-reception signal of the present embodiment is different from the case of FIG. 11 in that, as shown in steps S70 to S72, the wavelength-variable filter 16 is set to the $\lambda1$ filter, and the $\lambda1$ light-reception signal group consisting of a plurality of light-reception signals is obtained and stored in the memory 38 as a working memory, followed by the calculation of the $\lambda1$ light-reception signal I1 from the $\lambda1$ light-reception signal group. The registration process of the reference light-reception signal of the present embodiment is also different in that, as shown in steps S73 to S75, the wavelength-variable filter 16 is set to the $\lambda2$ filter, and the $\lambda2$ light-reception signal including a plurality of light-reception signals is obtained, followed by the calculation of the $\lambda2$ amount of light received I2 from the $\lambda2$ light-reception signal.

[Third Embodiment]

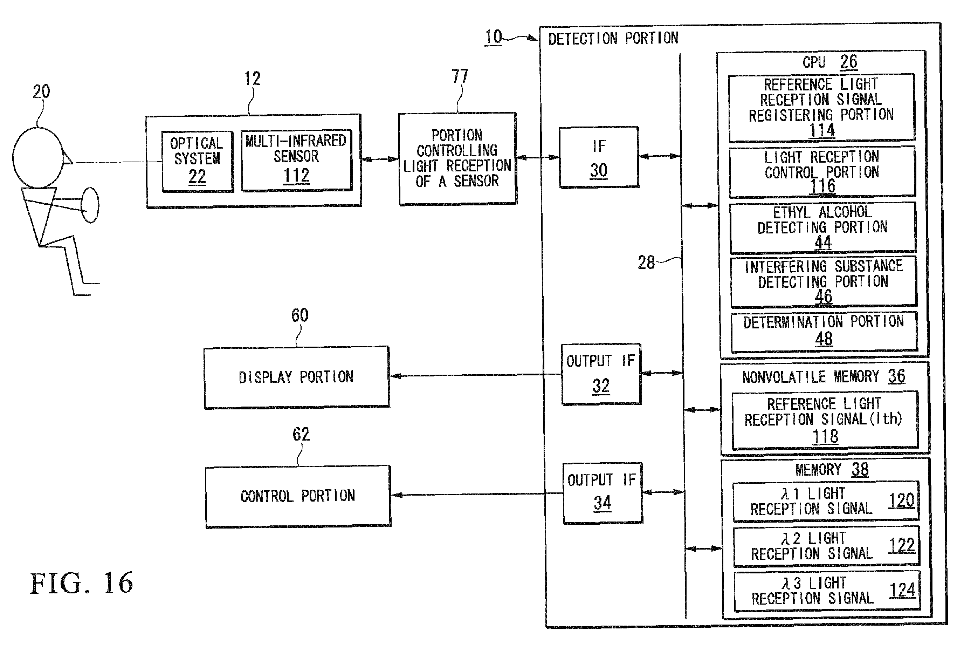

FIG. 16 is a block diagram showing the fourth embodiment of the ethyl alcohol-detecting device of the present invention, which is mounted on a vehicle.

In the infrared camera 12 of the present embodiment, a multi-infrared sensor 112 including a built-in filter is provided as well as the optical system 22.

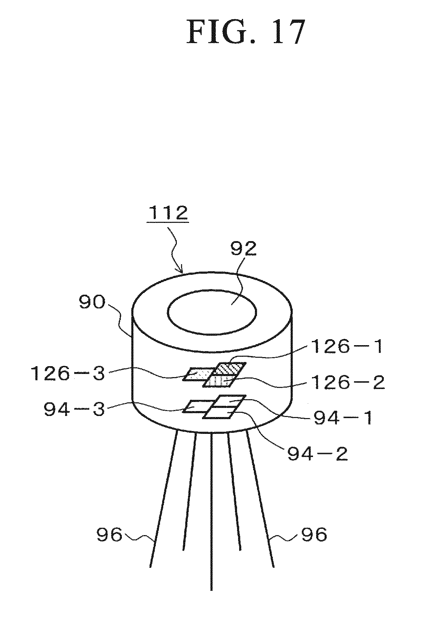

FIG. 17 is a view perspectively showing the internal structure the multi-infrared sensor 112.

In this multi-infrared sensor 112, for example, three infrared light-receiving elements 94-1 to 94-3 are arranged inside the case 90 where the window 92 is provided in the light-reception side, and in front of the light-receiving elements, a $\lambda1$ filter 126-1, a $\lambda2$ filter 126-2, and a $\lambda3$ filter 126-3 are arranged.

The $\lambda1$ filter 126-1 is a filter that transmits the spectral absorption wavelength of ethyl alcohol, $\lambda1=2.77\ \mu m$. The $\lambda2$ filter 126-2 is a filter that transmits a wavelength $\lambda2=3.28\ \mu m$ which is intrinsic to menthol as an interfering substance. The $\lambda3$ filter 126-3 is a filter that transmits a wavelength $\lambda3=5.88\ \mu m$ which is intrinsic to stearic acid as an interfering substance.

In this manner, according to the multi-infrared sensor 112 having a configuration in which the filters and the infrared light-receiving elements are combined with each other in one-to-one correspondence, the filter control conducted by the wavelength-variable filter 16 as in the case of the embodiments shown in FIGS. 8 and 2 is not necessary. Moreover, the multi-infrared sensor 112 itself can directly detect light-reception signals corresponding to the wavelengths $\lambda1$, $\lambda2$, and $\lambda3$ steadily. Therefore, the device configuration can be simplified, and the process performed by the CPU 26 is simplified, whereby it is possible to reduce a load applied to the process.

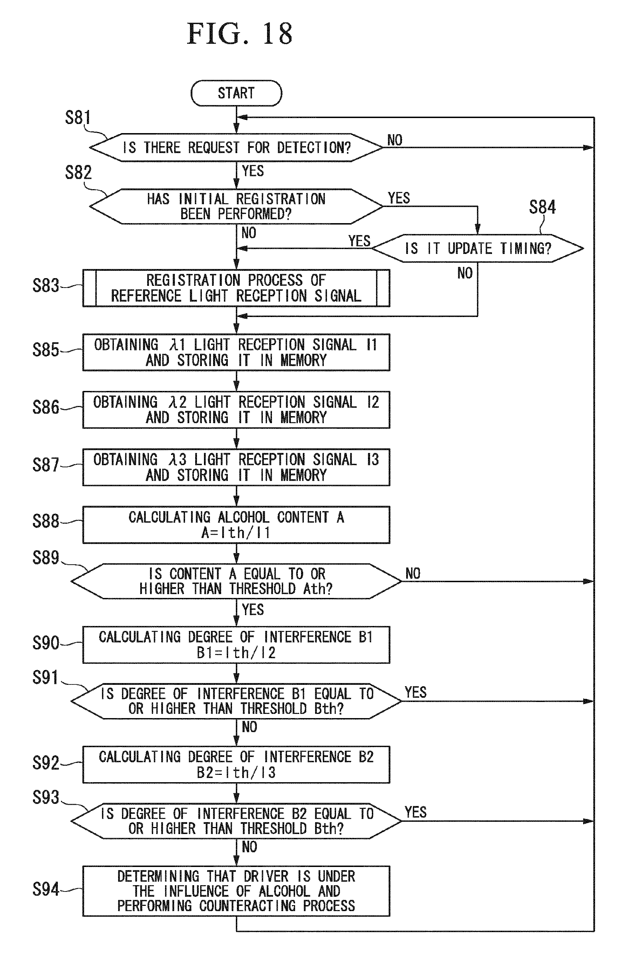

FIG. 18 is a flowchart showing the ethyl alcohol detection process in the present embodiment.

As shown in FIG. 18, steps S81 to S83 are the same as steps S54 to S56 shown in FIG. 14.

On the other hand, in the subsequent steps S85 to S87, the wavelength-variable filter 16 is not provided, and a filter is built in the multi-infrared sensor 112 itself as shown in FIG. 17. Accordingly, the $\lambda1$ light-reception signal I1, the $\lambda2$ light-reception signal I2, and the $\lambda3$ light-reception signal I3 can be directly obtained and stored in the memory 38.

Thereafter, in step S88, the ethyl alcohol content A is calculated. If the content A is equal to or higher than the threshold Ath in step S89, the degree of interference B 1 is calculated in step S90. If the degree of interference B1 is less than the threshold Bth in step S91, a degree of interference B2 is calculated in step S92. On the other hand, if the degree of interference B2 is less than the threshold Bth in step S93, it is determined that alcohol has been detected. Therefore, in step S94, it is determined that the driver is in a state under the influence of alcohol and a process for counteracting this state is performed.

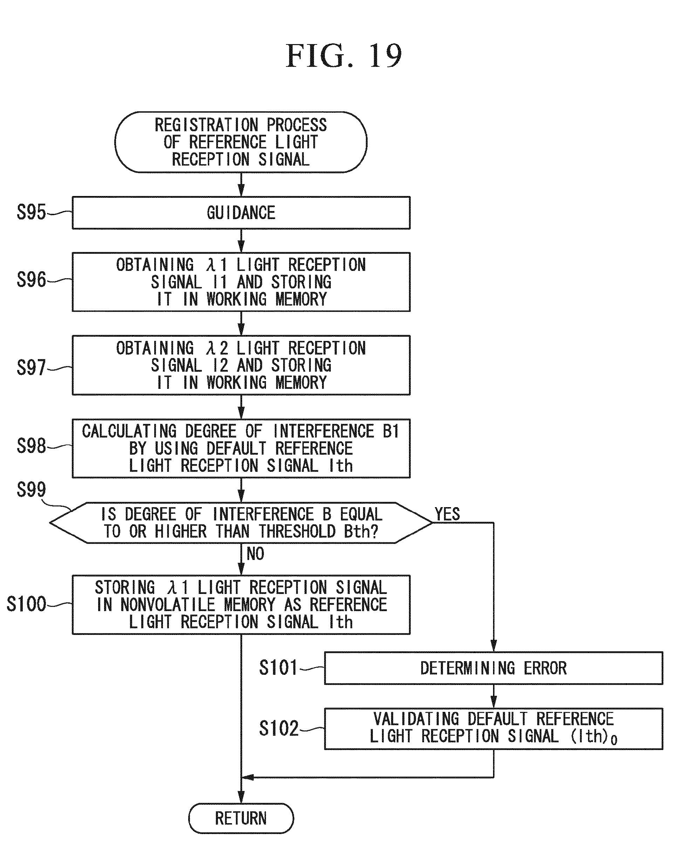

FIG. 19 is a flowchart showing the detail of the registration process of the reference amount of light received performed in step S83 of FIG. 18.

In this registration process of the reference amount of light received, guidance for the registration process is provided in step S95. Thereafter, in steps S96 and S97, from the infrared light-receiving elements 94-1 and 94-2 respectively corresponding to the $\lambda1$ filter 126-1 and the $\lambda2$ filter 126-2 of the multi-infrared sensor 112, the $\lambda1$ light-reception signal I1 and the $\lambda2$ light-reception signal I2 are directly obtained and stored in the memory 38 as a working memory.

In the subsequent step S98, the degree of interference B is calculated from the Formula (2) using the default reference light-reception signal Ith and the $\lambda2$ light-reception signal I2. In step S99, if the degree of interference B is less than the threshold Bth, the process proceeds to step S100. In step S100, since there is no influence exerted by an interfering substance, the $\lambda1$ light-reception signal is stored in the nonvolatile memory 36 as the reference light-reception signal Ith. On the other hand, if the degree of interference B is equal to or higher than the threshold Bth in step S99, it is determined that there is an error, in step S 101. In step S 102, the default reference light-reception signal $(\text{Ith})_0$ is validated, and the process returns to the main routine of FIG. 18.

In the respective embodiments described above, filter-switching performed by a wavelength-variable filter or a mechanical filter-switching unit was exemplified. However, in addition to this, a spectroscope using an analytical grid may be used so as to measure images or amounts of light received in a target spectral band.

The present invention also includes appropriate modifications that do not impair the object and advantages of the present invention. In addition, the present invention is not limited only to the numerical values shown in the above embodiments.

Industrial Applicability

According to the present invention, it is possible to provide a maintenance-free ethyl alcohol-detecting device which is usable for preventing driving under the influence of alcohol by detecting ethyl alcohol in the blood from infrared that a driver radiates.

Reference Signs List

| | |
|---|---|
| 10: | detecting portion |
| 12: | infrared camera |
| 14: | camera control portion |
| 16: | wavelength-variable filter |
| 18: | filter-driving portion |
| 20: | driver |
| 22: | optical system |
| 24: | CCD imaging element |
| 26: | CPU |
| 28: | bus |
| 30, 32, 34: | IF |
| 36: | nonvolatile memory |
| 38: | memory |
| 40: | reference image-registering unit |
| 42: | imaging control unit |
| 44: | ethyl alcohol-detecting portion |
| 46: | interfering substance-detecting portion |
| 48: | determination portion |
| 50: | reference image |
| 52: | reference amount of light received |
| 54: | characteristic area extracted image |
| 56: | λ1 image |
| 58: | λ2 image |
| 60: | display portion |
| 62: | control portion |
| 64: | object lens |
| 66: | imaging lens |
| 72-1 to 72-3: | characteristic-extracting areas |
| 74: | filter-switching unit |
| 76-1, 126-1: | λ1 filters |
| 76-2, 126-2: | λ2 filters |
| 77: | portion controlling light-reception of a sensor |
| 78: | infrared sensor |
| 80, 100, 114: | reference light-reception signal registration process portions |
| 82, 102, 116: | light-reception control portions |
| 84, 104: | reference light-reception signals |
| 86: | λ1 light-reception signal |
| 88: | λ2 light-reception signal |
| 90: | case |

| 92: | window |
| 94, 94-1 to 94-3: | infrared-detecting elements |
| 96: | lead |
| 98, 112: | multi-infrared sensors |
| 106, 120: | λ1 light-reception signal groups |
| 108, 122: | λ2 light-reception signal groups |
| 124: | λ3 light-reception signal group |
| 126-3: | λ3 filter |

**Claims**

1. An ethyl alcohol-detecting device comprising:

an imaging element that has sensitivity to an infrared wavelength band;
an optical system that forms an image of a subject on the imaging element;
a memory and a nonvolatile memory that store the image captured by the imaging element;
a first filter that selectively transmits infrared light of a first wavelength band including an absorption wavelength of ethyl alcohol released from the subject;
a second filter that selectively transmits infrared light of a second wavelength band including a wavelength which is another absorption wavelength of an interfering substance having an absorption wavelength as same as that of the ethyl alcohol released from the subject in the first wavelength band and is a wavelength other than the absorption wavelength of the ethyl alcohol;
an imaging control unit that controls the imaging element to capture a first subject image formed by passing through the first filter and a second subject image formed by passing through the second filter and that stores the images in the memory;
a reference image-registering unit that stores the first subject image in the nonvolatile memory as a reference image, at the time of setting a registration mode;
an ethyl alcohol-detecting portion that calculates an ethyl alcohol content corresponding to the ethyl alcohol concentration, based on the first subject image stored in the memory and the reference image stored in the nonvolatile memory;
an interfering substance-detecting portion that calculates a degree of interference corresponding to the concentration of the interfering substance, based on the second subject image stored in the memory and the reference image stored in the nonvolatile memory; and
a determination portion that determines that ethyl alcohol has been detected when the ethyl alcohol content is equal to or higher than a first predetermined threshold and the degree of interference is less than a second predetermined threshold, and determines that ethyl alcohol has not been detected when the ethyl alcohol content is equal to or higher than the first predetermined threshold and the degree of interference is equal to or higher than the second predetermined threshold.

2. The ethyl alcohol-detecting device according to Claim 1,
wherein the first filter selectively transmits infrared light in a wavelength band including 2.77 μm or 3.37 μm as the first wavelength band; and
the second filter selectively transmits infrared light in a wavelength band including 3.28 μm as the second wavelength band when the interfering substance is menthol, and selectively transmits infrared light in a wavelength band including 5.88 μm as the second wavelength band when the interfering substance is stearic acid.

3. The ethyl alcohol-detecting device according to Claim 1,
wherein the reference image-registering unit calculates a reference amount of light received as the sum or average of amounts of light received by respective pixels constituting the reference image and stores the reference amount of light received in the nonvolatile memory;
the ethyl alcohol-detecting portion calculates the ethyl alcohol content based on an amount of light received by the first subject that is calculated as the sum or average of amounts of light received by respective pixels of the first subject image and the reference amount of light received which is stored in the nonvolatile memory; and
the interfering substance-detecting portion calculates the degree of interference based on an amount of light received by the second subject that is calculated as the sum or average of the amounts of light received by respective pixels of the second subject image and the reference amount of light received which is stored in the nonvolatile memory.

**4.** The ethyl alcohol-detecting device according to Claim 3,
wherein the ethyl alcohol-detecting portion calculates the ethyl alcohol content as (i) a subtracted value that is obtained by subtracting the amount of light received by the first subject from the reference amount of light received, or a divided value that is obtained by dividing the reference amount of light received by the amount of light received by the first subject, (ii) a value obtained by multiplying the subtracted value or the divided value by a first predetermined coefficient, or (iii) the sum or average of squared error values of the amount of light received by the first subject and the reference amount of light received; and
the interfering substance-detecting portion calculates the degree of interference as (iv) a subtracted value obtained by subtracting the amount of light received by the second subject from the reference amount of light received or a divided value obtained by dividing the reference amount of light received by the amount of light received by the second subject, (v) a value obtained by multiplying the subtracted value or the divided value by a second predetermined coefficient, or (vi) the sum or average of squared error values of the amount of light received by the second subject and the reference amount of light received.

**5.** An ethyl alcohol-detecting device comprising:

an infrared sensor that has at least one infrared light-receiving element;
an optical system that forms an image of a subject on the infrared light-receiving sensor;
a memory and a nonvolatile memory that store a light-reception signal received by the infrared sensor;
a first filter that selectively transmits infrared light of a first wavelength band including an absorption wavelength of ethyl alcohol released from the subject;
a second filter that selectively transmits infrared light of a second wavelength band including a wavelength which is another absorption wavelength of an interfering substance having an absorption wavelength as same as that of the ethyl alcohol released from the subject in the first wavelength band and is a wavelength other than the absorption wavelength of the ethyl alcohol;
a light-reception control portion that detects a first light-reception signal of the subject passing through the first filter and received by the infrared sensor and a second light-reception signal of the subject passing through the second filter and received by the infrared sensor, and stores the signals in the memory;
a reference light-reception signal registering portion that stores the first light-reception signal in the nonvolatile memory as a reference light-reception signal, at the time of setting a registration mode;
an ethyl alcohol-detecting portion that calculates an ethyl alcohol content corresponding to the ethyl alcohol concentration, based on the first light-reception signal stored in the memory and the reference light-reception signal stored in the nonvolatile memory;
an interfering substance-detecting portion that calculates a degree of interference corresponding to the concentration of the interfering substance, based on the second light-reception signal stored in the memory and the reference light-reception signal stored in the nonvolatile memory; and
a determination portion that determines that ethyl alcohol has been detected when the ethyl alcohol content is equal to or higher than a first predetermined threshold and the degree of interference is less than a second predetermined threshold, and determines that ethyl alcohol has not been detected when the ethyl alcohol content is equal to or higher than the first predetermined threshold and the degree of interference is equal to or higher than the second predetermined threshold.

**6.** The ethyl alcohol-detecting device according to Claim 5,
wherein the first filter selectively transmits infrared light in a wavelength band including 2.77 $\mu$m or 3.37 $\mu$m as the first wavelength band; and
the second filter selectively transmits infrared light in a wavelength band including 3.28 $\mu$m as the second wavelength band when the interfering substance is menthol, and selectively transmits infrared light in a wavelength band including 5.88 $\mu$m as the second wavelength band when the interfering substance is stearic acid.

**7.** The ethyl alcohol-detecting device according to Claim 5,
wherein the ethyl alcohol-detecting portion calculates the ethyl alcohol content based on the first light-reception signal stored in the memory and the reference light-reception signal stored in the nonvolatile memory; and
the interfering substance-detecting portion calculates the degree of interference based on the second light-reception signal stored in the memory and the reference light-reception signal stored in the nonvolatile memory.

**8.** The ethyl alcohol-detecting device according Claim 7,
wherein the ethyl alcohol-detecting portion calculates the ethyl alcohol content as (i) a subtracted value that is obtained by subtracting the first light-reception signal from the reference light-reception signal, or a divided value that is obtained by dividing the reference light-reception signal by the first light-reception signal, (ii) a value obtained by multiplying the subtracted value or the divided value by a first predetermined coefficient, or (iii) the sum or average of squared error values of the first light-reception signal and the reference light-reception signal; and

the interfering substance-detecting portion calculates the degree of interference as (iv) a subtracted value obtained by subtracting the second light-reception signal from the reference light-reception signal or a divided value obtained by dividing the reference light-reception signal by the second light-reception signal, (v) a value obtained by multiplying the subtracted value or the divided value by a second predetermined coefficient, or (vi) the sum or average of squared error values of the second light-reception signal and the reference light-reception signal.

9. The ethyl alcohol-detecting device according to Claim 5,
wherein the infrared sensor further includes a sensor case that accommodates a combination of the first filter, at least a sheet of the second filter, and the infrared light-receiving element.

FIG. 1

DETECTION PORTION 10

CPU 26
- REFERENCE IMAGE REGISTERING PORTION 40
- IMAGING CONTROL PORTION 42
- ETHYL ALCOHOL DETECTING PORTION 44
- INTERFERING SUBSTANCE DETECTING PORTION 46
- DETERMINATION PORTION 48

NONVOLATILE MEMORY 36
- REFERENCE IMAGE 50
- REFERENCE AMOUNT OF LIGHT RECEIVED(Ith) 52
- CHARACTERISTIC AREA EXTRACTING IMAGE 54

MEMORY 38
- $\lambda 1$ IMAGE 56
- $\lambda 2$ IMAGE 58

OPTICAL SYSTEM 22
CCD IMAGING ELEMENT 24
CAMERA CONTROL PORTION 14
FILTER DRIVING PORTION 18
IF 30
OUTPUT IF 32
OUTPUT IF 34
DISPLAY PORTION 60
CONTROL PORTION 62

16
12
20
28

EP 2 484 284 A1

19

# FIG. 2

FILTER DRIVING PORTION

FROM 14

FIG. 3

EP 2 484 284 A1

# FIG. 4

FIG. 5

```
                        ┌──────────┐
                        │  START   │
                        └────┬─────┘
                             │
     S1 ─┐                   ▼
     ╱ IS THERE REQUEST FOR DETECTION? ╲──── NO ───┐
     ╲                                  ╱          │
              │ YES                                │
     S2 ─┐    ▼                                    │
     ╱ HAS INITIAL REGISTRATION ╲──── YES ──┐      │
     ╲    BEEN PERFORMED?        ╱          │  S4  │
              │ NO                          ▼      │
              │        ┌── YES ─╱ IS IT UPDATE TIMING? ╲──┤
              │        │        ╲                      ╱  │
              │        │                 │ NO             │
     S3 ─┐    ▼        │                                  │
   ┌─────────────────────────────────┐                   │
   │ REGISTRATION PROCESS OF AMOUNT OF│                   │
   │ LIGHT RECEIVED BY REFERENCE IMAGE│                   │
   └────────────┬────────────────────┘                   │
                │◄─────────────────────                   │
     S5 ─┐      ▼                                         │
   ┌─────────────────────────────────┐                   │
   │        SETTING λ1 FILTER         │                   │
   └────────────┬────────────────────┘                   │
     S6 ─┐      ▼                                         │
   ┌─────────────────────────────────┐                   │
   │     OBTAINING λ1 IMAGE AND       │                   │
   │       STORING IT IN MEMORY       │                   │
   └────────────┬────────────────────┘                   │
     S7 ─┐      ▼                                         │
   ┌─────────────────────────────────┐                   │
   │        SETTING λ2 FILTER         │                   │
   └────────────┬────────────────────┘                   │
     S8 ─┐      ▼                                         │
   ┌─────────────────────────────────┐                   │
   │     OBTAINING λ2 IMAGE AND       │                   │
   │       STORING IT IN MEMORY       │                   │
   └────────────┬────────────────────┘                   │
     S9 ─┐      ▼                                         │
   ┌─────────────────────────────────┐                   │
   │    EXTRACTING CHARACTERISTIC SITE│                   │
   └────────────┬────────────────────┘                   │
     S10 ─┐     ▼                                         │
   ┌─────────────────────────────────┐                   │
   │     CALCULATING AMOUNT OF LIGHT  │                   │
   │      RECEIVED I1 FROM λ1 IMAGE   │                   │
   └────────────┬────────────────────┘                   │
     S11 ─┐     ▼                                         │
   ┌─────────────────────────────────┐                   │
   │     CALCULATING AMOUNT OF LIGHT  │                   │
   │      RECEIVED I2 FROM λ2 IMAGE   │                   │
   └────────────┬────────────────────┘                   │
     S12 ─┐     ▼                                         │
   ┌─────────────────────────────────┐                   │
   │     CALCULATING ALCOHOL CONTENT A│                   │
   │            A=Ith/I1              │                   │
   └────────────┬────────────────────┘                   │
     S13 ─┐     ▼                                         │
     ╱ IS CONTENT A EQUAL TO OR ╲──── NO ────────────────┤
     ╲ HIGHER THAN THRESHOLD Ath? ╱                       │
              │ YES                                       │
     S14 ─┐   ▼                                           │
   ┌─────────────────────────────────┐                   │
   │  CALCULATING DEGREE OF INTERFERENCE B                │
   │            B=Ith/I2              │                   │
   └────────────┬────────────────────┘                   │
     S15 ─┐     ▼                                         │
     ╱ IS DEGREE OF INTERFERENCE B EQUAL TO ╲── YES ──────┤
     ╲ OR HIGHER THAN THRESHOLD Bth?        ╱             │
              │ NO                                        │
     S16 ─┐   ▼                                           │
   ┌─────────────────────────────────┐                   │
   │ DETERMINING THAT DRIVER IS UNDER │                   │
   │  THE INFLUENCE OF ALCOHOL AND    │                   │
   │ PERFORMING COUNTERACTING PROCESS │                   │
   └────────────┬────────────────────┘                   │
                └────────────────────────────────────────┘
```

**FIG. 6**

$$\boxed{\begin{array}{c}\text{REGISTRATION PROCESS OF}\\\text{AMOUNT OF LIGHT RECEIVED}\\\text{BY REFERENCE IMAGE}\end{array}}$$

S17 — GUIDANCE

S18 — SETTING $\lambda 1$ FILTER

S19 — CAPTURING $\lambda 1$ IMAGE AND STORING IT IN WORKING MEMORY

S20 — SETTING AREA OF CHARACTERISTIC SITE

S21 — CALCULATING AMOUNT OF LIGHT RECEIVED I1 OF $\lambda 1$ IMAGE FROM $\lambda 1$ IMAGE

S22 — SETTING $\lambda 2$ FILTER

S23 — CAPTURING $\lambda 2$ IMAGE AND STORING IT IN WORKING MEMORY

S24 — CALCULATING AMOUNT OF LIGHT RECEIVED I2 FROM $\lambda 2$ IMAGE

S25 — CALCULATING DEGREE OF INTERFERENCE B BY USING DEFAULT AMOUNT OF LIGHT RECEIVED Ith OF REFERENCE IMAGE

S26 — IS DEGREE OF INTERFERENCE B EQUAL TO OR HIGHER THAN THRESHOLD Bth? — YES

NO

S27 — STORING $\lambda 1$ AMOUNT OF LIGHT RECEIVED IN NONVOLATILE MEMORY AS REFERENCE AMOUNT OF LIGHT RECEIVED Ith

S29 — DETERMINING ERROR

S30 — VALIDATING DEFAULT AMOUNT OF LIGHT RECEIVED $(Ith)_0$ OF REFERENCE IMAGE

S28 — STORING CHARACTERISTIC AREA-SET IMAGE IN NONVOLATILE MEMORY

RETURN

# FIG. 7

FIG. 8

EP 2 484 284 A1

# FIG. 9

# FIG. 10

START

S31 — IS THERE REQUEST FOR DETECTION? →NO

↓YES

S32 — HAS INITIAL REGISTRATION BEEN PERFORMED? →YES

↓NO

S34 — IS IT UPDATE TIMING?

YES→

NO

S33 — REGISTRATION PROCESS OF REFERENCE LIGHT RECEPTION SIGNAL

S35 — SETTING $\lambda 1$ FILTER

S36 — OBTAINING $\lambda 1$ LIGHT RECEPTION SIGNAL I1 AND STORING IT IN MEMORY

S37 — SETTING $\lambda 2$ FILTER

S38 — OBTAINING $\lambda 2$ LIGHT RECEPTION SIGNAL I2 AND STORING IT IN MEMORY

S39 — CALCULATING ALCOHOL CONTENT A
$A = Ith/I1$

S40 — IS CONTENT A EQUAL TO OR HIGHER THAN THRESHOLD Ath? →NO

↓YES

S41 — CALCULATING DEGREE OF INTERFERENCE B
$B = Ith/I2$

S42 — IS DEGREE OF INTERFERENCE B EQUAL TO OR HIGHER THAN THRESHOLD Bth? →YES

↓NO

S43 — DETERMINING THAT DRIVER IS UNDER THE INFLUENCE OF ALCOHOL AND PERFORMING COUNTERACTING PROCESS

# FIG. 11

REGISTRATION PROCESS
OF REFERENCE LIGHT
RECEPTION SIGNAL

S44 — GUIDANCE

S45 — SETTING $\lambda 1$ FILTER

S46 — OBTAINING $\lambda 1$ LIGHT RECEPTION
SIGNAL I1 AND STORING
IT IN WORKING MEMORY

S47 — SETTING $\lambda 2$ FILTER

S48 — OBTAINING $\lambda 2$ LIGHT RECEPTION
SIGNAL I2 AND STORING
IT IN WORKING MEMORY

S49 — CALCULATING DEGREE OF INTERFERENCE B1
BY USING DEFAULT REFERENCE
LIGHT RECEPTION SIGNAL Ith

S50 — IS DEGREE OF INTERFERENCE B EQUAL
TO OR HIGHER THAN THRESHOLD Bth? — YES

NO

S51 — STORING $\lambda 1$ LIGHT RECEPTION SIGNAL
IN NONVOLATILE MEMORY AS REFERENCE
LIGHT RECEPTION SIGNAL Ith

S52 — DETERMINING ERROR

S53 — VALIDATING DEFAULT REFERENCE
LIGHT RECEPTION SIGNAL $(Ith)_0$

RETURN

FIG. 12

EP 2 484 284 A1

FIG. 13

# FIG. 14

```
                              ( START )
                                  │
                                  ▼
  S54 ───┐                                                    NO
         ◇ IS THERE REQUEST FOR DETECTION? ─────────────────────────────────┐
                                  │ YES                                       │
  S55 ───┐                                                    YES            │
         ◇ HAS INITIAL REGISTRATION ──────────────────┐                     │
            BEEN PERFORMED?                            │        S57          │
                                  │ NO        YES      ▼                      │
                                  │◄───────── ◇ IS IT UPDATE TIMING? ◇       │
                                  │                         │ NO             │
  S56 ───┌──────────────────────────────────┐              │                │
         │ REGISTRATION PROCESS OF REFERENCE │◄─────────────┘                │
         │     LIGHT RECEPTION SIGNAL        │                               │
         └──────────────────────────────────┘                               │
                                  │◄──────────────────────────────────       │
                                  ▼                                          │
  S58 ───┌──────────────────────────────────┐                               │
         │       SETTING λ1 FILTER           │                               │
         └──────────────────────────────────┘                               │
                                  ▼                                          │
  S59 ───┌──────────────────────────────────┐                               │
         │ OBTAINING λ1 LIGHT RECEPTION SIGNAL│                              │
         │  GROUP AND STORING IT IN MEMORY   │                               │
         └──────────────────────────────────┘                               │
                                  ▼                                          │
  S60 ───┌──────────────────────────────────┐                               │
         │       SETTING λ2 FILTER           │                               │
         └──────────────────────────────────┘                               │
                                  ▼                                          │
  S61 ───┌──────────────────────────────────┐                               │
         │ OBTAINING λ2 LIGHT RECEPTION SIGNAL│                              │
         │  GROUP AND STORING IT IN MEMORY   │                               │
         └──────────────────────────────────┘                               │
                                  ▼                                          │
  S62 ───┌──────────────────────────────────┐                               │
         │ CALCULATING AMOUNT OF LIGHT RECEIVED I1│                          │
         │  FROM λ1 LIGHT RECEPTION SIGNAL GROUP│                            │
         └──────────────────────────────────┘                               │
                                  ▼                                          │
  S63 ───┌──────────────────────────────────┐                               │
         │ CALCULATING AMOUNT OF LIGHT RECEIVED I2│                          │
         │  FROM λ2 LIGHT RECEPTION SIGNAL GROUP│                            │
         └──────────────────────────────────┘                               │
                                  ▼                                          │
  S64 ───┌──────────────────────────────────┐                               │
         │  CALCULATING ETHYL ALCOHOL CONTENT A│                             │
         │            A=Ith/I1               │                               │
         └──────────────────────────────────┘                               │
                                  ▼                                          │
  S65 ───┐                                                    NO            │
         ◇ IS CONTENT A EQUAL TO OR ───────────────────────────────────────┤
            HIGHER THAN THRESHOLD Ath?                                       │
                                  │ YES                                      │
  S66 ───┌──────────────────────────────────┐                               │
         │ CALCULATING DEGREE OF INTERFERENCE B│                             │
         │            B=Ith/I2               │                               │
         └──────────────────────────────────┘                               │
                                  ▼                                          │
  S67 ───┐                                                    YES           │
         ◇ IS DEGREE OF INTERFERENCE B EQUAL TO ─────────────────────────────┤
            OR HIGHER THAN THRESHOLD Bth?                                    │
                                  │ NO                                       │
  S68 ───┌──────────────────────────────────┐                               │
         │ DETERMINING THAT DRIVER IS UNDER  │                               │
         │   THE INFLUENCE OF ALCOHOL AND    │                               │
         │ PERFORMING COUNTERACTING PROCESS  │                               │
         └──────────────────────────────────┘                               │
                                  │                                          │
                                  └──────────────────────────────────────────┘
```

## FIG. 15

```
      REGISTRATION PROCESS
        OF REFERENCE LIGHT
        RECEPTION SIGNAL
```

S69 — GUIDANCE

S70 — SETTING $\lambda 1$ FILTER

S71 — OBTAINING $\lambda 1$ LIGHT RECEPTION SIGNAL GROUP AND STORING IT IN WORKING MEMORY

S72 — CALCULATING $\lambda 1$ AMOUNT OF LIGHT RECEIVED I1 FROM $\lambda 1$ LIGHT RECEPTION SIGNAL GROUP

S73 — SETTING $\lambda 2$ FILTER

S74 — OBTAINING $\lambda 2$ LIGHT RECEPTION SIGNAL GROUP AND STORING IT IN WORKING MEMORY

S75 — CALCULATING $\lambda 2$ AMOUNT OF LIGHT RECEIVED I2 FROM $\lambda 2$ LIGHT RECEPTION SIGNAL GROUP

S76 — CALCULATING DEGREE OF INTERFERENCE B BY USING DEFAULT REFERENCE LIGHT RECEPTION SIGNAL Ith

S77 — IS DEGREE OF INTERFERENCE B EQUAL TO OR HIGHER THAN THRESHOLD Bth? — YES

NO

S78 — STORING $\lambda 1$ LIGHT RECEPTION SIGNAL IN NONVOLATILE MEMORY AS REFERENCE LIGHT RECEPTION SIGNAL Ith

S79 — DETERMINING ERROR

S80 — VALIDATING DEFAULT LIGHT RECEPTION SIGNAL $(Ith)_0$ OF REFERENCE IMAGE

RETURN

EP 2 484 284 A1

FIG. 16

20

12

77

10 → DETECTION PORTION

OPTICAL SYSTEM 22 | MULTI-INFRARED SENSOR 112

PORTION CONTROLLING LIGHT RECEPTION OF A SENSOR

IF 30

CPU 26

REFERENCE LIGHT RECEPTION SIGNAL REGISTERING PORTION 114

LIGHT RECEPTION CONTROL PORTION 116

28

ETHYL ALCOHOL DETECTING PORTION 44

INTERFERING SUBSTANCE DETECTING PORTION 46

DETERMINATION PORTION 48

60

DISPLAY PORTION

OUTPUT IF 32

NONVOLATILE MEMORY 36

REFERENCE LIGHT RECEPTION SIGNAL(Ith) 118

62

CONTROL PORTION

OUTPUT IF 34

MEMORY 38

λ1 LIGHT RECEPTION SIGNAL 120

λ2 LIGHT RECEPTION SIGNAL 122

λ3 LIGHT RECEPTION SIGNAL 124

EP 2 484 284 A1

# FIG. 17

35

## FIG. 18

```
                    ( START )
                        │
S81         ┌───────────┴───────────┐        NO
        ◇── IS THERE REQUEST FOR DETECTION? ──────────────────────┐
            └───────────┬───────────┘                             │
S82                     │ YES                                      │
        ┌───────────────┴───────────────┐   YES                   │
    ◇── HAS INITIAL REGISTRATION         ────────────┐    S84      │
        BEEN PERFORMED?                              │             │
        └───────────────┬───────────────┘           ▼             │
                        │ NO           YES  ┌────────────────┐     │
                        ◄─────────────────── IS IT UPDATE TIMING? ◇│
                        │                   └────────┬───────┘     │
S83     ┌───────────────┴───────────────┐           │ NO          │
        │ REGISTRATION PROCESS OF        │           │             │
        │ REFERENCE LIGHT RECEPTION SIGNAL│◄──────────┘            │
        └───────────────┬───────────────┘                         │
S85     ┌───────────────┴───────────────┐                         │
        │ OBTAINING λ1 LIGHT RECEPTION SIGNAL I1 │                 │
        │ AND STORING IT IN MEMORY       │                         │
        └───────────────┬───────────────┘                         │
S86     ┌───────────────┴───────────────┐                         │
        │ OBTAINING λ2 LIGHT RECEPTION SIGNAL I2 │                 │
        │ AND STORING IT IN MEMORY       │                         │
        └───────────────┬───────────────┘                         │
S87     ┌───────────────┴───────────────┐                         │
        │ OBTAINING λ3 LIGHT RECEPTION SIGNAL I3 │                 │
        │ AND STORING IT IN MEMORY       │                         │
        └───────────────┬───────────────┘                         │
S88     ┌───────────────┴───────────────┐                         │
        │ CALCULATING ALCOHOL CONTENT A  │                         │
        │ A=Ith/I1                       │                         │
        └───────────────┬───────────────┘                         │
S89     ┌───────────────┴───────────────┐   NO                    │
    ◇── IS CONTENT A EQUAL TO OR         ─────────────────────────┤
        HIGHER THAN THRESHOLD Ath?       │                         │
        └───────────────┬───────────────┘                         │
                        │ YES                                      │
S90     ┌───────────────┴───────────────┐                         │
        │ CALCULATING DEGREE OF INTERFERENCE B1 │                  │
        │ B1=Ith/I2                      │                         │
        └───────────────┬───────────────┘                         │
S91     ┌───────────────┴───────────────┐   YES                   │
    ◇── IS DEGREE OF INTERFERENCE B1 EQUAL TO ────────────────────┤
        OR HIGHER THAN THRESHOLD Bth?    │                         │
        └───────────────┬───────────────┘                         │
                        │ NO                                       │
S92     ┌───────────────┴───────────────┐                         │
        │ CALCULATING DEGREE OF INTERFERENCE B2 │                  │
        │ B2=Ith/I3                      │                         │
        └───────────────┬───────────────┘                         │
S93     ┌───────────────┴───────────────┐   YES                   │
    ◇── IS DEGREE OF INTERFERENCE B2 EQUAL TO ────────────────────┤
        OR HIGHER THAN THRESHOLD Bth?    │                         │
        └───────────────┬───────────────┘                         │
                        │ NO                                       │
S94     ┌───────────────┴───────────────┐                         │
        │ DETERMINING THAT DRIVER IS UNDER │                       │
        │ THE INFLUENCE OF ALCOHOL AND     │                       │
        │ PERFORMING COUNTERACTING PROCESS │                       │
        └───────────────┬───────────────┘                         │
                        └─────────────────────────────────────────┘
```

# FIG. 19

REGISTRATION PROCESS
OF REFERENCE LIGHT
RECEPTION SIGNAL

S95 — GUIDANCE

S96 — OBTAINING $\lambda 1$ LIGHT RECEPTION SIGNAL I1 AND STORING IT IN WORKING MEMORY

S97 — OBTAINING $\lambda 2$ LIGHT RECEPTION SIGNAL I2 AND STORING IT IN WORKING MEMORY

S98 — CALCULATING DEGREE OF INTERFERENCE B1 BY USING DEFAULT REFERENCE LIGHT RECEPTION SIGNAL Ith

S99 — IS DEGREE OF INTERFERENCE B EQUAL TO OR HIGHER THAN THRESHOLD Bth?

NO

YES

S100 — STORING $\lambda 1$ LIGHT RECEPTION SIGNAL IN NONVOLATILE MEMORY AS REFERENCE LIGHT RECEPTION SIGNAL Ith

S101 — DETERMINING ERROR

S102 — VALIDATING DEFAULT REFERENCE LIGHT RECEPTION SIGNAL $(Ith)_0$

RETURN

## INTERNATIONAL SEARCH REPORT

| | International application No. |
|---|---|
| | PCT/JP2009/005133 |

**A. CLASSIFICATION OF SUBJECT MATTER**
*A61B5/1455*(2006.01)i, *B60K28/06*(2006.01)i, *G08G1/14*(2006.01)i, *G08G1/16*
(2006.01)i

According to International Patent Classification (IPC) or to both national classification and IPC

**B. FIELDS SEARCHED**

Minimum documentation searched (classification system followed by classification symbols)
A61B5/1455, B60K28/06, G08G1/14, G08G1/16

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2009-187359 A (Hochiki Corp.),<br>20 August 2009 (20.08.2009),<br>paragraphs [0050] to [0079], [0118] to [0119]<br>(Family: none) | 1-9 |

☐ Further documents are listed in the continuation of Box C.    ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 November, 2009 (11.11.09) | 24 November, 2009 (24.11.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H08150853 B

- JP H08285688 B